(19) Europäisches Patentamt
European Patent Office
Office européen des brevets

(11) Publication number: **0 468 469 A2**

# (12) EUROPEAN PATENT APPLICATION

(21) Application number: **91112403.0**

(22) Date of filing: **24.07.91**

(51) Int. Cl.5: **C07D 207/08**, C07D 207/09, C07D 207/16, C07D 207/34, A61K 31/40

(30) Priority: **27.07.90 JP 197835/90**
**27.12.90 JP 418334/90**

(43) Date of publication of application:
**29.01.92 Bulletin 92/05**

(84) Designated Contracting States:
**AT BE CH DE DK ES FR GB IT LI NL SE**

(71) Applicant: **Japan Tobacco Inc.**
**4-12-62 Higashishinagawa**
**Shinagawa-ku, Tokyo 140(JP)**

Applicant: **YOSHITOMI PHARMACEUTICAL INDUSTRIES, LTD.**
**6-9, Hiranomachi 2-chome Chuo-ku**
**Osaka-shi Osaka 541(JP)**

(72) Inventor: **Koji, Kobayashi, c/o Pharm. Research Lab.**
**Japan Tobacco Inc., 6-2, Umegaoka, Midori-ku**
**Yokohama-shi, Kanagawa 227(JP)**
Inventor: **Kazuhiko, Nishii, c/o Pharm. Research Lab.**
**Japan Tobacco Inc., 6-2, Umegaoka, Midori-ku**
**Yokohama-shi, Kanagawa 227(JP)**
Inventor: **Kunio, Iwata, c/o Toxicology Research Laboratories**
**Japan Tobacco Inc., 23, Nakogi**
**Hatano-shi, Kanagawa 257(JP)**
Inventor: **Itsuo, Uchida, c/o Pharm. Research Lab.**
**Japan Tobacco Inc., 6-2, Umegaoka, Midori-ku**
**Yokohama-shi, Kanagawa 227(JP)**

(74) Representative: **von Kreisler, Alek, Dipl.-Chem. et al**
**Patentanwälte Von Kreisler-Selting-Werner, Deichmannhaus am Hauptbahnhof**
**W-5000 Köln 1(DE)**

## (54) Proline derivatives.

(57) Novel proline derivatives of the following formula

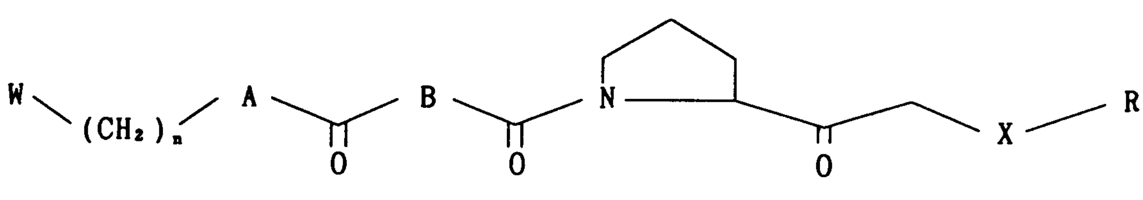

wherein:
A is -O-, -NH-,

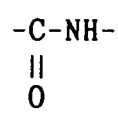

EP 0 468 469 A2

or absent;
B is

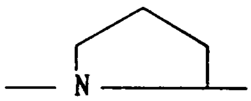, 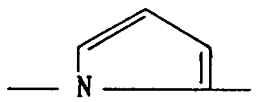, 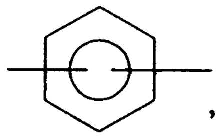, 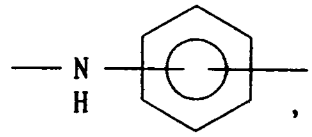,

$-(CH_2)_k-$ or

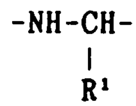

where k is an integer of 1 to 3, $R^1$ is hydrogen atom or a lower alkyl having 1 to 5 carbon atoms;
W is

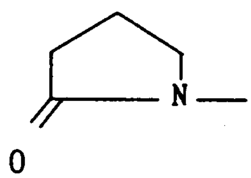, 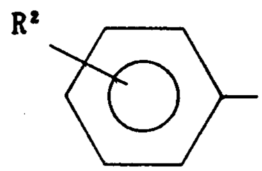

or $CH_3$- where $R^2$ is hydrogen atom, halogen atom or a lower alkoxy;
X is -S-, -SO-, $-SO_2-$, -O- or -NH-;
R is

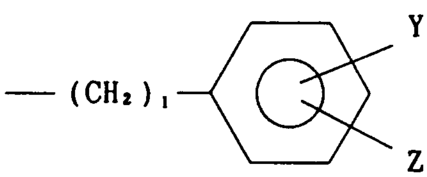

or a lower alkyl having 1 to 5 carbon atoms where 1 is an integer of 0 to 3, Y and Z are the same or different and each is hydrogen atom, halogen atom, a lower alkyl having 1 to 5 carbon atoms which may be substituted by fluorine atom, amino, nitro, hydroxyl or a lower alkoxy, and Y and Z may combinedly form a saturated or unsaturated 5- or 6-membered ring; and
n is an integer of 1 to 6, which specifically inhibit prolyl endopeptidase activity and can be used for the prevention and/or treatment of dementia and amnesia as agents which act directly on the central symptoms of dementia.

FIELD OF THE INVENTION

The present invention relates to novel proline derivatives possessing prolyl endopeptidase-inhibitory activities, method of production thereof and pharmaceutical use thereof.

BACKGROUND OF THE INVENTION

Along with the arrival of an aging society, the medical treatment of the senile disease has been drawing much attention. Above all, senile dementia has become a serious social problem, and various developments have been made in an attempt to provide new pharmaceuticals to cope with the problem. The conventional agents for the treatment of amnesia and dementia have been named obscurely as cerebral circulation improving agents, cerebral metabolism activator or cerebral function improving agents according to their action mechanisms. While they are effective for the improvement of peripheral symptoms such as depression, emotional disturbances, abnormal behavior, etc., they do not show definite effects on the central symptoms of dementia, such as memory disorder, disorientation, or the like. Thus, the development of medicaments which can offer dependable action and effect on these symptoms is earnestly desired.

In the meantime, prolyl endopeptidase; EC, 3.4.21.26 is an enzyme known to act on peptides containing proline and to specifically cleave out the carboxyl side of the proline. Further, this enzyme is known to act on neurotransmitters such as thyrotropin-releasing hormone (TRH), substance P and neurotensin, as well as on vasopressin which is presumably concerned with learning and memory process, resulting in decomposition and inactivation of them.

In view of the foregoing findings, a compound possessing inhibitory activity on prolyl endopeptidase is expected to suppress decomposition and inactivation of vasopressin, etc., thereby suggesting a potential application thereof to the treatment and prevention of amnesia and dementia as an efficacious medicament which exhibits direct action on the central symptoms of dementia [See *Seikagaku*, *55*, 831 (1983); *FOLIA PHARMACOL. JAPON*, *89*, 243 (1987); and J. Pharmacobio-Dyn., *10*, 730 (1987)] and also to suppress decomposition and inactivation of hormones and neurotransmitters such as TRH, substance P, neurotensin, etc., thereby improving various symptoms caused by the decomposition and inactivation of these substances.

From the motivation described above, there have been attempted to develop prolyl endopeptidase-inhibiting agents and various proline derivatives are described and disclosed in, for example, Japanese Patent Unexamined Publication Nos. 188317/1985, 148467/1987, 42475/1989 and 28149/1990.

SUMMARY OF THE INVENTION

Based on the findings mentioned above, the present inventors have conducted intensive studies to find a compound which possesses an amino acid, specifically a proline residue as a fragment and specifically inhibits the prolyl endopeptidase activity, and found that novel proline derivatives of the formula (I) possessed specific and strong prolyl endopeptidase-inhibitory activity, which resulted in completion of the invention.

An object of the invention is to provide a novel compound possessing specific and strong prolyl endopeptidase-inhibitory activities.

Another object of the invention is to provide pharmaceutical composition such as prolyl endopeptidase-inhibitors.

Still another object of the invention is to provide production methods of the aforementioned novel compound.

The present invention relates to novel proline derivatives of the following formula (I)

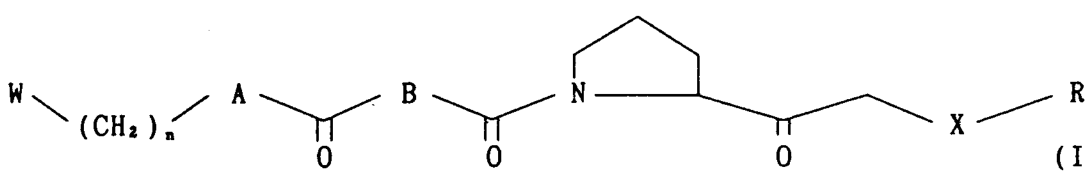

(I)

wherein:
A is -O-, -NH-,

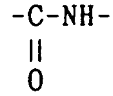

or absent;
B is

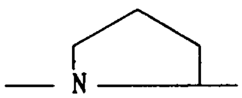, 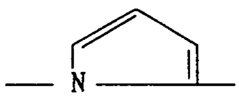, 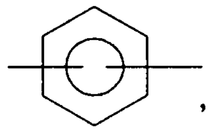, 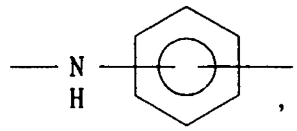,

$-(CH_2)_k-$ or

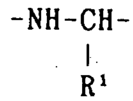

where k is an integer of 1 to 3, $R^1$ is hydrogen atom or a lower alkyl having 1 to 5 carbon atoms;
W is

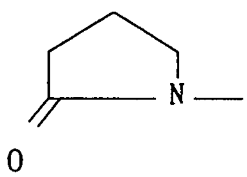, 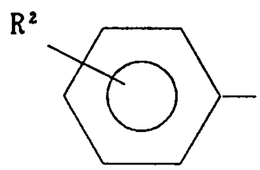

or $CH_3$- where $R^2$ is hydrogen atom, halogen atom or a lower alkoxy;
X is -S-, -SO-, $-SO_2-$, -O- or -NH-;
R is

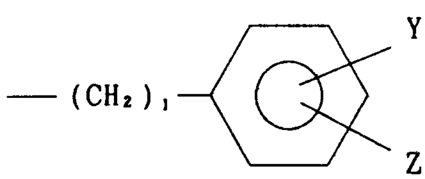

or a lower alkyl having 1 to 5 carbon atoms where 1 is an integer of 0 to 3, Y and Z are the same or different and each is hydrogen atom, halogen atom, a lower alkyl having 1 to 5 carbon atoms which may be substituted by fluorine atom, amino, nitro, hydroxyl or a lower alkoxy, and Y and Z may combinedly form a saturated or unsaturated 5- or 6-membered ring; and
n is an integer of 1 to 6,
and to pharmaceutical compositions useful as a prolyl endopeptidase-inhibitor containing the novel proline derivative (I) as an active ingredient.

Further, the present invention relates to methods for producing novel proline derivatives of the following formula (I)

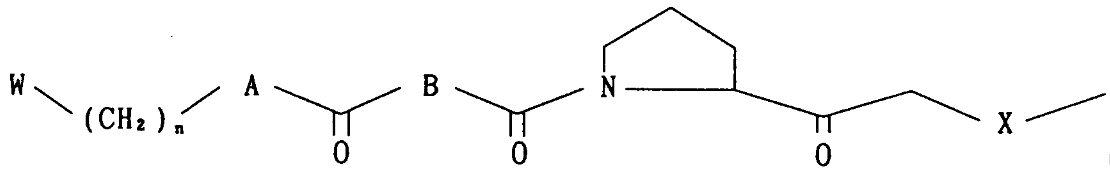

(I)

wherein W, A, B, X, R and n are as defined above, which comprise subjecting a compound of the formula

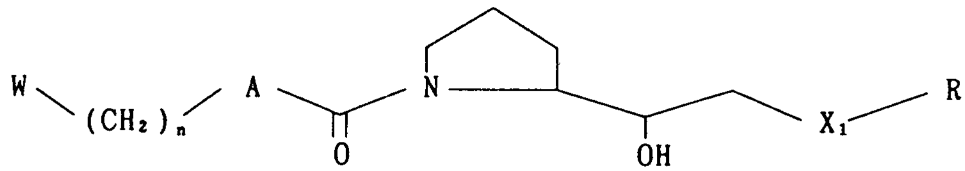

wherein $X_1$ is S, SO or $SO_2$, and W, A, R are as defined above, or a compound of the formula

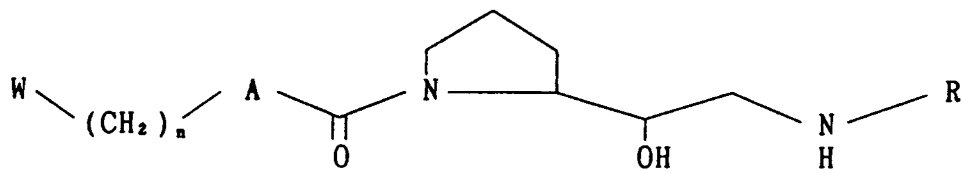

wherein W, A and R are as defined above, or an optically active compound of the formula

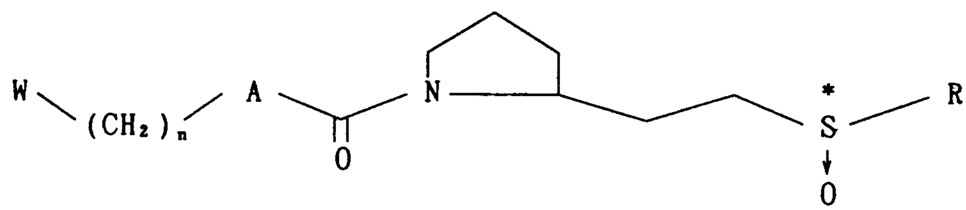

wherein W, A and R are as defined above, to oxidation; or reacting an optically active compound of the formula

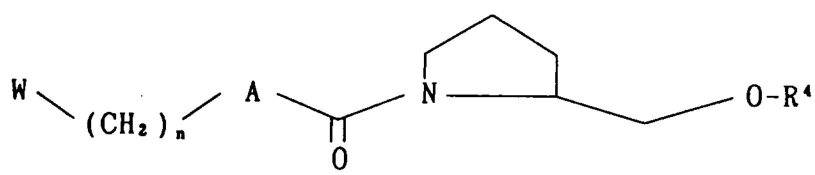

wherein $R^4$ is a carbonyl protecting group and W, A and R are as defined above, with a compound of the formula

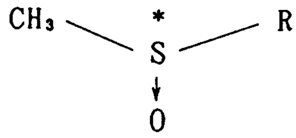

wherein R is as defined above.

DETAILED DESCRIPTION OF THE INVENTION

Various substituents used in the present invention are defined as follows.

Halogen atom means chlorine, bromine, fluorine and iodine; lower alkyl having 1 to 5 carbon atoms means straight or branched hydrocarbon chains having 1 to 5 carbon atoms, which are exemplified by methyl, ethyl, propyl, isopropyl, butyl, sec-butyl and tert-butyl; and lower alkoxy means methoxy, ethoxy, propoxy, isopropoxy, butoxy, sec-butoxy, tert-butoxy, and so on.

The novel proline derivatives of the formula (I) of the invention can be produced, for example, by the reaction processes shown below.

Production of Compound I

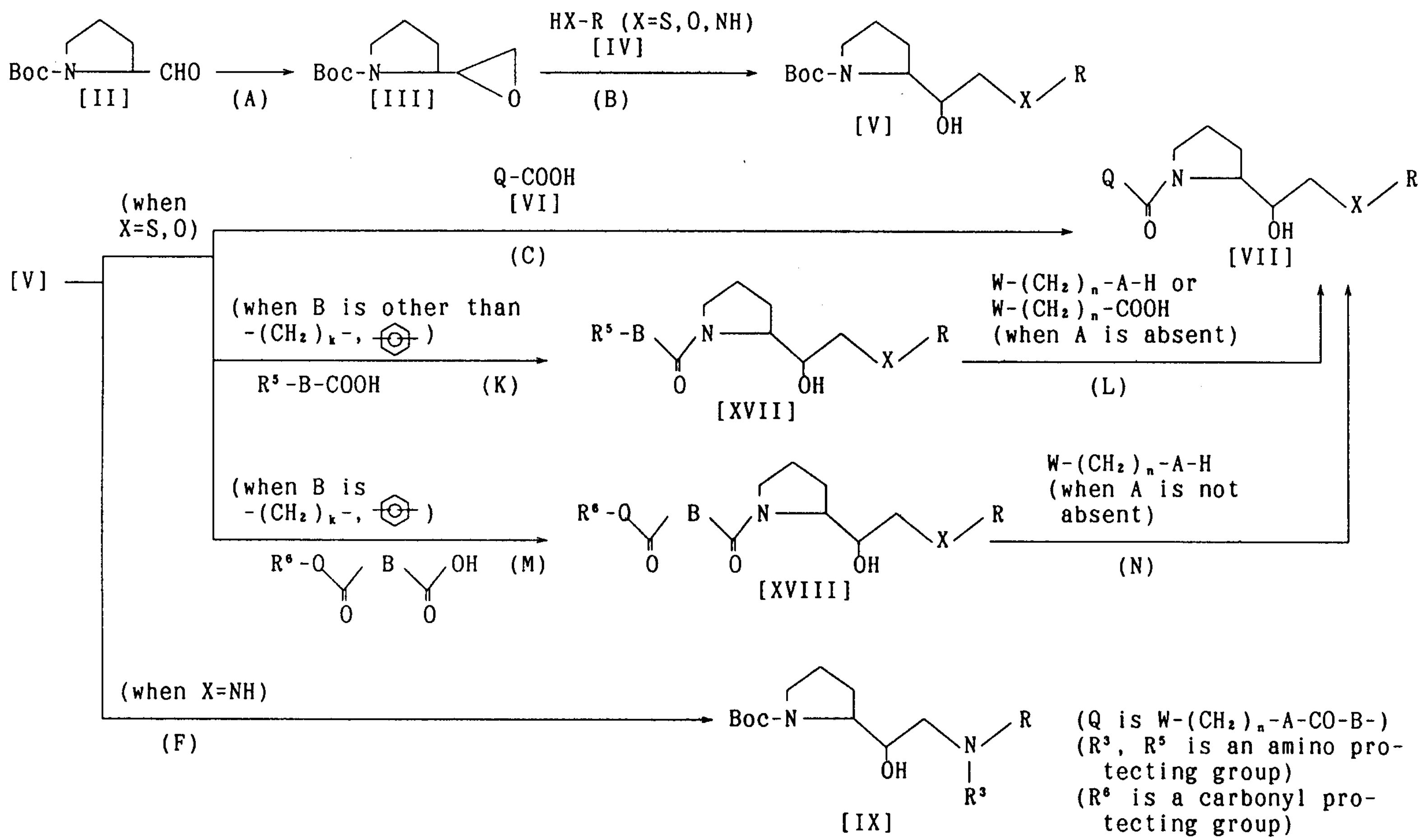

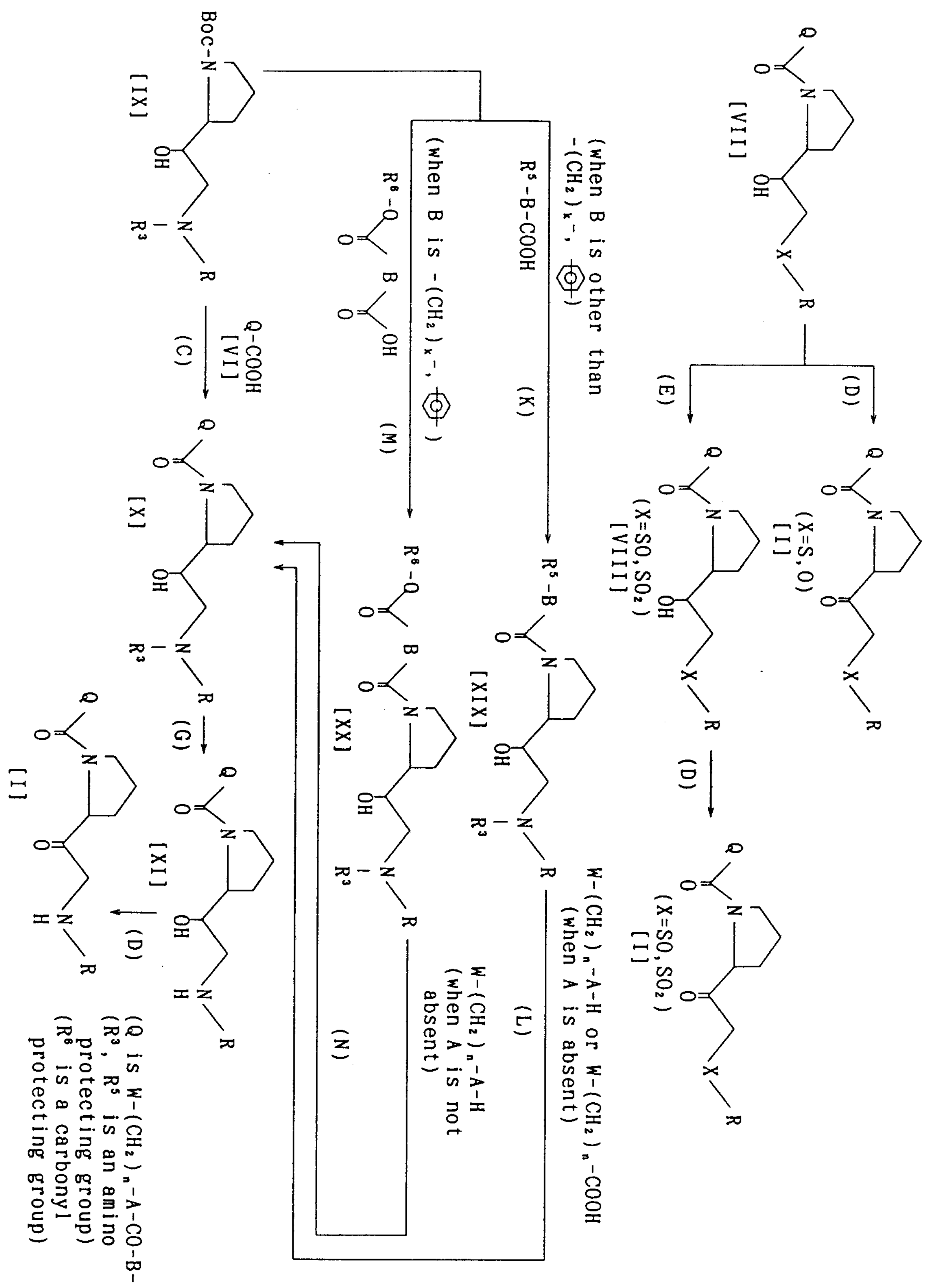


Hereunder follow detailed descriptions in respect of each reaction processes. The symbols A, B, W, X, R, k and n are as defined above.

<u>Reaction</u> <u>(A)</u>

A compound of the formula (II) is reacted with a sulfur ylid derived from trimethylsulfonium iodide or trimethyloxosulfonium iodide in the presence of a strong base, to obtain an epoxide of the formula (III). This reaction can be conducted, for example, by obtaining a sulfur ylid from the above-mentioned sulfonium salt using n-butyllithium or sodium hydridedimethyl sulfoxide in an inert solvent such as tetrahydrofuran, dioxane and hexane, followed by reaction of the obtained sulfur ylid with a compound (II). The reaction temperature is between -70 °C and reflux temperature, preferably between -10 °C and room temperature.

Reaction (B)

A compound of the formula (III) is reacted with a compound (IV) of the formula HX-R (X = S, O, NH) in a suitable solvent in the presence or absence of a base, to obtain an alcohol compound of the formula (V). Detailedly speaking, a reaction with HX-R wherein X is sulfur atom is conducted in the presence of a tertiary amine such as triethylamine and N-methylmorpholine in a solvent such as methanol, dioxane and dimethylformamide. A reaction with HX-R wherein X is an oxygen atom is conducted using sodium methoxide in methanol, or using sodium hydride in a solvent such as dioxane and dimethylformamide to convert HO-R to anion ⊖O-R, followed by reaction with (III). A reaction with HX-R wherein X is NH is conducted in a solvent such as methanol and dioxane. The reaction temperature is between room temperature and reflux temperature in each case described above.

Reaction (C)

t-Butoxycarbonyl (Boc) group which is an amino protecting one for an intermediate of the formula (V) and (IX) is removed by a known method, after which the compounds are subjected to a condensation reaction with a compound of the formula (VI) to give compounds (VII) and (X), respectively.

Removal of the Boc group can be conducted by a known method, such as by acid treatment of an intermediate of the formula (V) or (IX) with the use of hydrobromic acid/acetic acid, hydrochloric acid/dioxane, formic acid, hydrochloric acid/acetic acid, trifluoroacetic acid, etc. at a temperature between -30 °C and 70 °C, preferably between 0 °C and 30 °C.

Then, the thus-obtained Boc-removed compound is subjected to a condensation reaction with a compound (VI) to give an amino acid derivative (VII) or (X).

A method known per se can be employed for this peptide formation. Examples thereof include methods such as N,N'-dicyclohexylcarbodiimide (DCC) method, activated ester method, mixed acid anhydride method, and so on. The reaction proceeds in an inert solvent at a temperature between 0 °C and under heating. Examples of suitable solvents include chloroform, diethyl ether, dimethylformamide, ethyl acetate, dichloromethane, tetrahydrofuran, or the like.

In the activated ester method, peptide bonds are formed by reacting the above-mentioned compound (VI) with p-nitrophenol, thiophenol, p-nitrothiophenol, N-hydroxysuccinimide, etc. in an inert solvent in the presence of DCC, thereby affording an activated ester (e.g. an ester with N-hydroxysuccinimide), and with or without isolation, further reacting the same with the above-mentioned Boc-removed compound in an inert solvent at a temperature between 0 °C and 40 °C.

In the mixed anhydride method, peptide bonds are formed by reacting the above-mentioned compound (VI) with an acid halide (e.g. pivaloyl chloride, tosyl chloride, oxalyl chloride) or an acid derivative (e.g. ethyl chloroformate, isobutyl chloroformate) in an inert solvent in the presence of a tertiary amine (e.g. pyridine, triethylamine) at a temperature between 0 °C and 40 °C, thereby affording a mixed acid anhydride, and further reacting the same with the Boc-removed compound mentioned above at a temperature between 0 °C and 40 °C.

In the DCC method, the desired peptide bonds are formed by reacting the above-mentioned Boc-removed compound and (VI) in an inert solvent in the presence or absence of the tertiary amine mentioned above such as triethylamine, or with or without addition of a suitable additive [e.g. 1-hydroxybenzotriazole (HOBt), N-hydroxy-5-norbornen-2,3-dicarboximide (HONB)] with DCC as a condensing agent.

Reaction (D)

The object compound (I) is prepared by oxidation of an alcohol compound of the formula (VII), (VIII) or (XI) with a suitable oxidizing agent.

This reaction can be conducted in an inert solvent such as benzene, dichloromethane and dimethylformamide using pyridinium chlorochromate or pyridinium dichromate in the presence or absence of molecular sieves at a temperature between 0 °C and reflux temperature, preferably between 0 °C and room temperature; or in an inert solvent such as dichloromethane in the presence of oxalyl chloride and triethylamine using dimethyl sulfoxide at a temperature between -80 °C and room temperature, preferably between -80 °C and 0 °C; or in the presence of pyridine, trifluoroacetic acid and dimethylsulfoxide using DCC with or without co-existence of an inert solvent such as benzene at a temperature between 0 °C and room temperature; or with or without co-existence of an inert solvent such as benzene in the presence of a tertiary amine such as triethylamine and dimethyl sulfoxide, using sulfur trioxide-pyridine complex at a temperature between -10 °C and room temperature.

Reaction (E)

A sulfoxide compound or a sulfone compound of the formula (VIII) is obtained by oxidizing a compound of the formula (VII) wherein X is a sulfur atom with a per acid such as m-chloroperbenzoic acid. For example, a sulfoxide compound and a sulfone compound can be obtained by oxidation in an inert solvent such as dichloromethane, chloroform and benzene using an equivalent and 2 equivalents of m-chloroperbenzoic acid, respectively, at a temperature between -20 °C and reflux temperature, preferably between 0 °C and room temperature.

Reaction (F)

A compound (IX) is obtained by introducing an amino protecting group $R^3$ to a compound of the formula (V) wherein X is NH by a method known in peptide synthesis.

While there are many examples for an amino protecting group $R^3$, those stable against acid treatment for removing Boc group for converting (IX) to (X) should be used, which are exemplified by formyl and trifluoroacetyl which are acyl type protecting groups, and 9-fluorenylmethoxycarbonyl, methylsulfonylethyloxycarbonyl which are urethane type protecting groups. Introduction of these protecting groups can be achieved by a known method (Izumiya, et al., ***Pepuchido Gosei no Kiso to Jikken,*** Maruzen). For example, introduction of trifluoroacetyl group can be conducted by a reaction of (V) with ethyl trifluoroacetate in a solvent such as methanol in the presence of a tertiary amine such as triethylamine at a temperature between 0 °C and room temperature, preferably at room temperature. In this reaction, $R^3$ may be introduced after protection of free hydroxyl group with an appropriate protecting group, if necessary.

Reaction (G)

A compound (XI) is obtained by removing an amino protecting group $R^3$ from a compound of the formula (X).

While the method for removing the amino protecting group $R^3$ varies depending on the kind of the protecting group, a known method in peptide synthesis can be employed. Such removal is mainly achieved by catalytic reduction or base treatment in respect of the aforementioned Reaction (F). For example, removal of trifluoroacetyl group is carried out in a solvent such as methanol using potassium carbonate, sodium carbonate or ammonia as a base at a temperature between 0 °C and reflux temperature, preferably at room temperature.

Known or new intermediates of the formula (VI) as described are to be discussed in the following.

An intermediate Q-COOH is in more detail expressed by the following formula

$W-(CH_2)_n-A-CO-B-COOH$

(wherein A, B, W and n are as defined above), and when unavailable in the market, the compound can be produced, for example, as in the following.

Reaction ① $W-(CH_2)_n-A-CO-V + H-B-COOMe$
$\rightarrow W-(CH_2)_n-A-CO-B-COOMe$
$\rightarrow W-(CH_2)_n-A-CO-B-COOH$

Reaction ② $W-(CH_2)_n-A-H + V-CO-B-COOMe$
$\rightarrow W-(CH_2)_n-A-CO-B-COOMe$
$\rightarrow W-(CH_2)_n-A-CO-B-COOH$

(wherein V is a chlorine atom or a hydroxyl group. Note that methyl ester was used in the above reactions, to which it is not limited, and benzyl ester, etc. can be also used.)

Particularly, the above Reaction ① is effective when A is oxygen atom or absent and B is

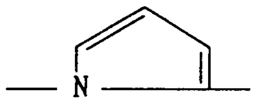 , 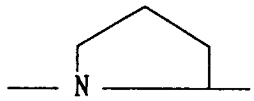 , 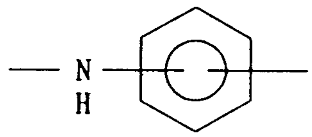

or

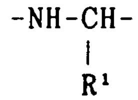 ,

and Reaction ② is useful when A is -NH- or -CONH- and B is

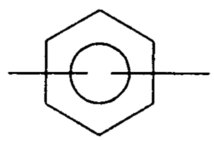

or $-(CH_2)_k-$. These reactions are explained in detail in the following.

Reaction ①

An amino compound of the formula H-B-COOMe is reacted with a compound of the formula $W-(CH_2)_n-A-CO-Cl$ in a solvent such as dichloromethane, chloroform, dioxane and water in the presence of a tertiary amine such as triethylamine and N-methylmorpholine, or sodium hydroxide at a temperature between 0 °C and room temperature; or reacted with a compound of the formula $W-(CH_2)_n-A-CO-OH$ using a peptide bond forming reaction as detailedly described in Reaction (C) above. Then, a compound of the formula $W-(CH_2)_n-A-CO-B-COOMe$ thus obtained is hydrolyzed with a base such as sodium hydroxide and lithium hydroxide at a temperature between 0 °C and room temperature by a known method.

Reaction ②

An amino (or amide) compound of the formula $W-(CH_2)_n-A-H$ is reacted with acid chloride of the formula Cl-CO-B-COOMe in a solvent such as dichloromethane, chloroform, dioxane and water in the presence of a tertiary amine such as triethylamine and N-methylmorpholine or sodium hydroxide at a temperature between 0 °C and room temperature; or reacted with a carboxylic acid of the formula HO-CO-B-COOMe using the peptide bond forming reaction as detailedly described in the above Reaction (C). Then, the thus-obtained compound of the formula $W-(CH_2)_n-A-CO-B-COOMe$ is hydrolyzed in the same manner as in Reaction ①.

Preparation of compounds (VII) and (X) conducted by condensation of compounds (V) and (IX), respectively with a compound (VI) may be replaced by the following stepwise reactions. That is, when B is other than $-(CH_2)_k-$ and

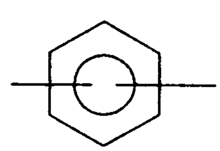 ,

a compound of the formula (XVII) can be obtained by condensation of a compound of the formula $R^5$-B-COOH (wherein $R^5$ is a suitable amino protecting group) with a compound (V) in the same manner as in Reaction (C), which is shown in Reaction (K). Then the amino protecting group $R^5$ is removed from the compound of the formula (XVII) by a known method as shown in Reaction (L), after which the compound is reacted with a compound of the formula $W-(CH_2)_n-A-H$ (wherein A is O, NH or CONH) or a compound of the

formula W-$(CH_2)_n$-COOH (wherein A is absent) to give the object compound (VII). In the former, for example, W-$(CH_2)_n$-A-H is reacted with phosgene, diphosgene, carbonyldiimidazole, or the like in a suitable solvent such as dioxane and tetrahydrofuran in the presence of a tertiary amine such as triethylamine at a temperature between -20 °C and room temperature, followed by reaction with a deprotected compound of (XVII). If necessary, a free hydroxyl group of compound (XVII) may be in advance protected with a suitable protecting group. When A is a single bond as in the latter, the method described in Reaction (C) above is employed wherein a deprotected compound of the formula (XVII) is condensed with W-$(CH_2)_n$-COOH.

When B is -$(CH_2)_k$- or

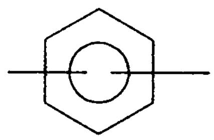,

a compound of the formula (XVIII) is obtained by condensation of a compound of the formula $R^6$-O-CO-B-COOH (wherein $R^6$ is a suitable carbonyl protecting group) with a compound (V) in the same manner as in Reaction (C), which is shown in Reaction (M). Then, the carbonyl protecting group $R^6$ is removed from the compound of the formula (XVIII) by a known method as shown in Reaction (N), after which the compound is reacted with a compound of the formula W-$(CH_2)_n$-A-H to give the object compound (VII). Concretely stated, the reaction can be conducted by esterification using W-$(CH_2)_n$-OH by a known method, amide bond forming reaction described in the above Reaction (C) usig W-$(CH_2)_n$-$NH_2$, or conversion of free carboxylic acid as obtained by deprotection ($R^6$) of the compound (XVIII) into acid chloride or active ester by a known method, followed by reaction with W-$(CH_2)_n$-$NH_2$. If necessary, a free hydroxyl group of compound (XVIII) may be in advance protected with a suitable protecting group. As regards preparation of a compound (X), when B is other than -$(CH_2)_k$- or

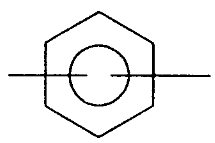,

a compound of the formula (X) can be obtained by converting a compound of the formula (IX) to a compound of the formula (XIX) in the same manner as in the above Reaction (K), followed by Reaction (L) as described. When B is -$(CH_2)_k$- or

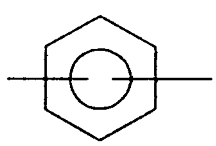,

a compound of the formula (X) can be obtained by converting a compound of the formula (IX) to a compound of the formula (XX) by Reaction (M), followed by Reaction (N) as described.

The compounds of the formula (I) thus obtained can be isolated and purified from the reaction mixture by a conventional method selected *ad libitum* according to the purpose, which has been usually employed in the field of synthetic organic chemistry. For example, column chromatography, solvent extraction, recrystallization, etc. can be used.

The isolation and purification may be conducted at every reaction or upon completion of a few reactions.

A series of compounds mentioned above possess 1 to 3 asymmetric center(s) in each molecule. In the present invention, the configuration of each asymmetric center may be R or S, and also a mixture of such optical isomers is encompassed in the invention. Optically active substances can be obtained respectively by employing an optically active compound as a starting material, or by purification of the obtained mixture of diastereomers by means of column chromatography, recrystallization, etc.

An optically active sulfinyl compound of the formula (I)

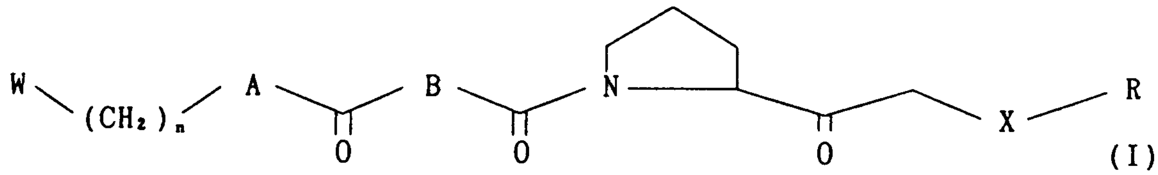

(I)

can be synthetically produced by the following steps.

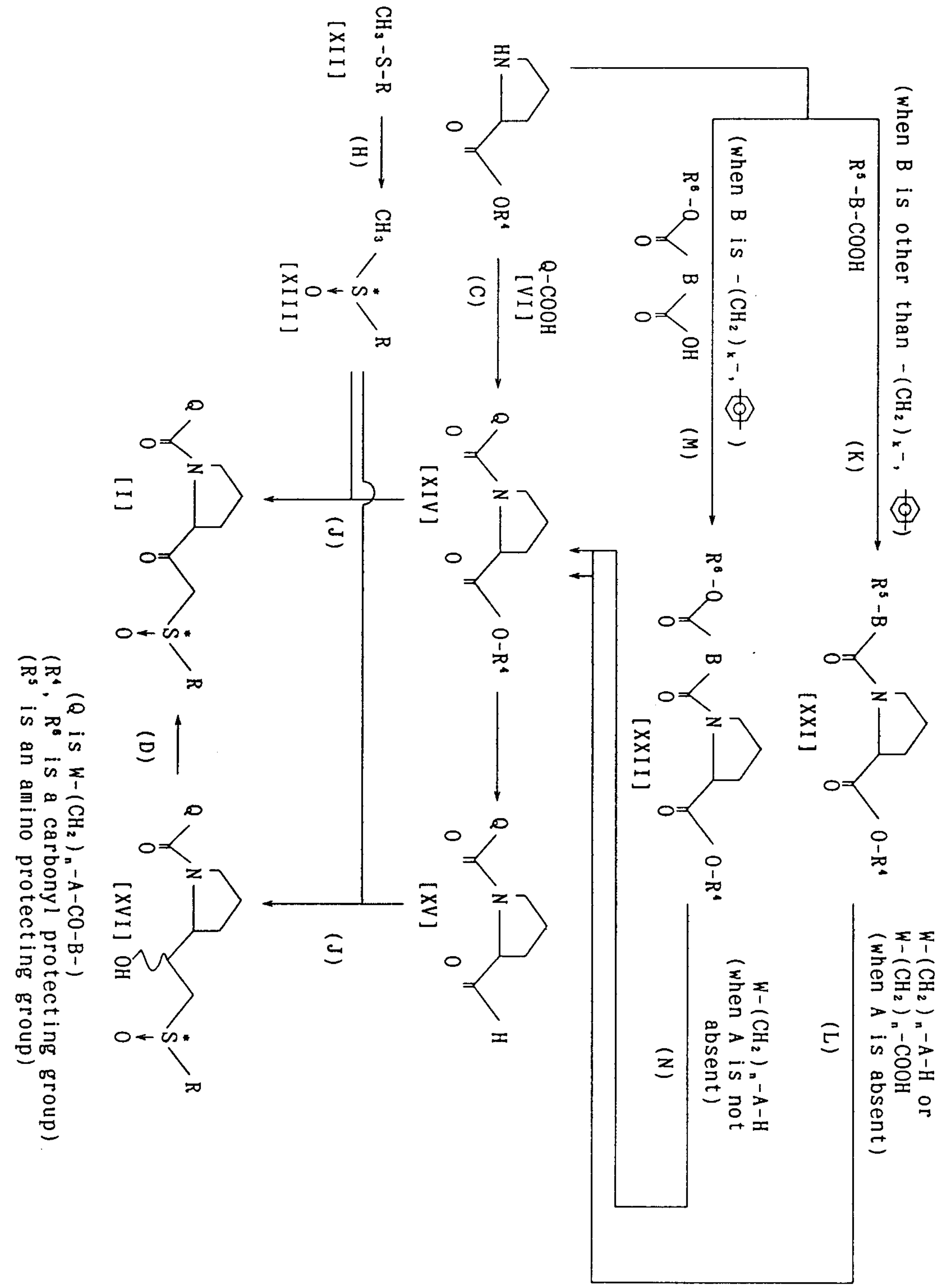

Each reaction step is detailedly described in the following, wherein A, B, W, R and n are as defined above, and Reaction (C), (D), (K), (L), (M) and (N) are as described above.

Reaction (H)

A compound of the formula (XII) is subjectd to asymmetric oxidation by a literature method [P. Pitchen et al., J. Am. Chem. Soc., *106*, 8188-8193 (1984); S. H. Zhao et al.. Tetrahedron, *43*, 5135-5144 (1987), etc.] to give an optically active sulfoxide of the formula (XIII). This reaction is conducted, for example, in a solvent such as dichloromethane, 1,2-dichloroethane in the presence of titanium tetraisopropoxide, optically active diethyl tartarate and water using t-butyl hydroperoxide or cumene hydroperoxide at a temperature of 0 °C or below, preferably at a temperature between -40 °C and -20 °C.

Reaction (J)

An optically active sulfoxide of the formula (XIII) is treated with a base to convert into the corresponding ylid which is then condensed with an ester (XIV) or an aldehyde (XV) to give an optically active sulfinyl compound of the formula (I) or (XVI). This reaction can be carried out by, for example, reacting an optically active sulfoxide (XIII) in an inert organic solvent such as tetrahydrofuran and dioxane using a base such as n-butyllithium and lithium diisopropylamide at a temperature between -78 °C and room temperature, preferably 0 °C or below to generate the corresponding ylid which is then subjected to condensation reaction with an ester (XIV) or an aldehyde (XV) at a temperature between -78 °C and room temperature, preferably between -78 °C and 20 °C.

When the compounds of the present invention are used as a pharmaceutical, they are administered systemically or locally, orally or parenterally.

While the dose varies depending on age, weight, symptom, treatment effect, method of administration, etc., the compounds of the present invention can be administered orally at 1 mg to 500 mg per administration per adult in a single unit dosage or in divided doses daily, or at 0.2 mg to 100 mg per administration per adult once to several times daily.

The compounds of the invention are administered in the form of solid compositions and liquid compositions for oral administration, or injections, suppositories, etc. for parenteral administration.

The solid compositions for oral administration include tablets, pills, capsules, powders, granules, etc. In the solid compositions, at least one active substance is mixed with at least one pharmaceutically acceptable inactive diluent, and excipients, binding agents, rublicants, degrading agents, dissolution enhancing agents, stabilizing agents, and so on may be also contained, if necessary. Tablets and pills may be applied with an enteric coating, if necessary. Capsules include hard and soft capsules.

The liquid compositions for oral administration include solutions, emulsions, suspensions, syrups and elixirs. These liquid compositions contain inactive diluents generally used and may further contain adjuvants such as wetting agents and suspending agents, sweetners, flavors, aromatic agents and preservatives.

The injections for parenteral administration include sterile aqueous or non-aqueous solutions, suspensions and emulsions. At least one active substance is used with at least one inactive aqueous diluent or inactive non-aqueous diluent in admixture, and other adjuvants such as preservatives, wetting agents, emulsifiers, dispersibles, stabilizing agents and dissolution enhancing agents may be also contained. They are normally sterilized by filtration (bacteria-retaining filter, etc. ), mixing with sterilizers or gamma irradiation, or they are, subsequent to such treatments, prepared into solid compositions by lyophilization and diluted before use with sterile water or sterile diluents for injection.

The present invention is hereinbelow described in detail by way of examples.

The abbreviations used in the examples respectively mean the following:

| | |
|---|---|
| DMF | dimethylformamide |
| DMSO | dimethyl sulfoxide |
| THF | tetrahydrofuran |
| HOBt | 1-hydroxybenzotriazole |
| DCC | N,N'-dicyclohexylcarbodiimide |
| $^1$H NMR | proton nuclear magnetic resonance spectra |
| CI-MS | chemical ionization mass spectrometry |
| FAB-MS | fast atom bombardment mass spectrometry |
| EI-MS | electron ionization mass spectra |

Example 1

(2S)-1-(N-Benzyloxycarbonyl-L-prolyl)-2-[(phenylthio)acetyl]pyrrolidine (Compound 1)

A) (2S)-1-(t-Butoxycarbonyl)-2-(1,2-epoxyethyl)pyrrolidine

DMSO (20 ml) was added to sodium hydride (60% NaH, 1.55 g), and the mixture was stirred at 70° C for 1 hour. The reaction mixture was cooled to room temperature and THF (20 ml) was added. The mixture was cooled to -5° C, and thereto was dropwise added trimethylsulfonium iodide (7.90 g) in DMSO (30 ml) over 3 minutes. After stirring for 1 minute, t-butoxycarbonyl-L-prolinal (5.13 g) in THF (15 ml) was added quickly, followed by stirring at room temperature for 1 hour. The reaction mixture was poured into ice water (300 ml) and extracted with chloroform. The organic layer was washed with saturated brine, dried over sodium sulfate and concentrated. The residue was purified by silica gel column chromatography (eluate : hexane-ethyl acetate) to give two kinds of diastereomers of (2S)-1-(t-butoxycarbonyl)-2-(1,2-epoxyethyl)-pyrrolidine, 3.12 g of its less polar compound and 1.28 g of its more polar compound. (Configuration of the epoxy moiety of the both unidentified.) The less polar compound was used in the following reactions.

B) (2S)-1-(t-Butoxycarbonyl)-2-[1-hydroxy-2-(phenylthio)ethyl]pyrrolidine

To (2S)-1-(t-butoxycarbonyl)-2-(1,2-epoxyethyl)pyrrolidine (3.00 g) in methanol (100 ml) were added thiophenol (1.5 ml) and triethylamine (2.0 ml), followed by 2 hours' stirring under reflux. The reaction mixture was concentrated and the residue was purified by silica gel column chromatography (eluate : hexane-ethyl acetate) to give 4.14 g of (2S)-1-(t-butoxycarbonyl)-2-[1-hydroxy-2-(phenylthio)ethyl]pyrrolidine.

C) (2S)-1-(N-Benzyloxycarbonyl-L-prolyl)-2-[1-hydroxy-2-(phenylthio)ethyl]pyrrolidine

4N Hydrochloric acid-dioxane (57 ml) was added to (2S)-1-(t-butoxycarbonyl)-2-[1-hydroxy-2-(phenylthio)ethyl]pyrrolidine (4.90 g), and the mixture was stirred at room temperature for 1 hour. The reaction mixture was concentrated to dryness and the residue was dissolved in DMF (100 ml), to which were added known N-benzyloxycarbonyl-L-proline (3.79 g), N-methylmorpholine (1.54 g) and HOBt (3.08 g). The mixture was cooled to -25° C, then added with DCC (3.13 g), and stirred at a temperature between -25° C and 0° C for 3 hours and at room temperature for 16 hours. The resultant dicyclohexylurea was filtered off and the filtrate was concentrated. A solution of the residue in ethyl acetate was washed with a saturated aqueous solution of sodium bicarbonate and saturated brine successively, dried over magnesium sulfate, and cencentrated. The residue was purified by silica gel column chromatography (eluate : chloroform-methanol) to give 6.56 g of (2S)-1-(N-Benzyloxycarbonyl-L-prolyl)-2-[1-hydroxy-2-(phenylthio)-ethyl]pyrrolidine.

D) (2S)-1-(N-Benzyloxycarbonyl-L-prolyl)-2-[(phenylthio)acetyl]pyrrolidine

(2S)-1-(N-Benzyloxycarbonyl-L-prolyl)-2-[1-hydroxy-2-(phenylthio)ethyl]pyrrolidine (908 mg) was dissolved in DMSO (3 ml) and benzene (3 ml), and thereto were added pyridine (162 μl), trifluoroacetic acid (78 μl) and DCC (1.24 g) in order, and t he mixture was stirred at room temperature for 1 hour. Ethyl acetate (50 ml) was added to the reaction mixture, and oxalic acid (540 mg) in methanol (5 ml) was added thereto, followed by stirring for 30 minutes. Water (50 ml) was added to the reaction mixture and the resultant dicyclohexylurea was filtered off. The filtrate was washed with 1N hydrochloric acid, a saturated aqueous solution of sodium bicarbonate and saturated brine in order, and dried over magnesium sulfate, followed by concentration. The residue was purified by silica gel column chromatography (eluate : chloroform-methanol) to give 714 mg of (2S)-1-(N-benzyloxycarbonyl-L-prolyl)-2-[(phenylthio)acetyl]-pyrrolidine (See Table 1).

Example 2

(2S)-1-(N-Benzyloxycarbonyl-L-prolyl)-2-[(phenylsulfinyl)acetyl]pyrrolidine (Compound 2)

A) (2S)-1-(N-Benzyloxycarbonyl-L-prolyl)-2-[1-hydroxy-2-(phenylsulfinyl)ethyl]pyrrolidine

To (2S)-1-(N-benzyloxycarbonyl-L-prolyl)-2-[1-hydroxy-2-(phenylthio)ethyl]pyrrolidine (908 mg) as obtained in Example 1-C) in dichloromethane (30 ml) was added m-chloroperbenzoic acid (380 mg), followed by 2 hours' stirring under ice-cooling. The reaction mixture was diluted with chloroform, washed with ice-

cooled 10% sodium sulfite, a saturated aqueous solution of sodium bicarbonate and saturated brine in order, and dried over magnesium sulfate, follwed by concentration. The residue was purified by silica gel column chromatography (eluate : chloroform-methanol) to give 895 mg of (2S)-1-(N-benzyloxycarbonyl-L-prolyl)-2-[1-hydroxy-2-(phenylsulfinyl) ethyl]pyrrolidine.

B) (2S)-1-(N-Benzyloxycarbonyl-L-prolyl)-2-[(phenylsulfinyl)acetyl]pyrrolidine

(2S)-1-(N-Benzyloxycarbonyl-L-prolyl)-2-[1-hydroxy-2-(phenylsulfinyl)ethyl]pyrrolidine (850 mg) was subjected to DMSO-DCC oxidation as in Example 1-D) to give 672 mg of (2S)-1-(N-benzyloxycarbonyl-L-prolyl)-2-[(phenylsulfinyl)acetyl]pyrrolidine (See Table 1).

Example 3

(2S)-1-(N-Benzyloxycarbonyl-L-prolyl)-2-[(phenylsulfonyl)acetyl]pyrrolidine (Compound 3)

A) (2S)-1-(N-Benzyloxycarbonyl-L-prolyl)-2-[1-hydroxy-2-(phenylsulfonyl)ethyl]pyrrolidine

m-Chloroperbenzoic acid (440 mg) was added to (2S)-1-(N-benzyloxycarbonyl-L-prolyl)-2-[1-hydroxy-2-(phenylthio)ethyl]pyrrolidine (516 mg) as obtained in Example 1-C) in dichloromethane (30 ml), followed by stirring at room temperature for 3 hours. The reaction mixture was treated and purified in the same manner as in Example 2-A) to give 540 mg of (2S)-1-(N-benzyloxycarbonyl-L-prolyl)-2-[1-hydroxy-2-(phenylsulfonyl)-ethyl]pyrrolidine.

B) (2S)-1-(N-Benzyloxycarbonyl-L-prolyl)-2-[(phenylsulfonyl)acetyl]pyrrolidine

(2S)-1-(N-Benzyloxycarbonyl-L-prolyl)-2-[1-hydroxy-2-(phenylsulfonyl)ethyl]pyrrolidine (500 mg) was subjected to DMSO-DCC oxidation as in Example 1-D) to give 412 mg of (2S)-1-(N-benzyloxycarbonyl-L-prolyl)-2-[(phenylsulfonyl)acetyl]pyrrolidine (See Table 1).

Example 4

(2S)-1-(N-Benzyloxycarbonyl-L-prolyl)-2-(phenoxyacetyl)pyrrolidine (Compound 4)

A) (2S)-1-(t-Butoxycarbonyl)-2-(1-hydroxy-2-phenoxyethyl)pyrrolidine

Phenol (1.50 g) and sodium methoxide (380 mg) were added to (2S)-1-(t-butoxycarbonyl)-2-(1,2-epoxyethyl)pyrrolidine (1.50 g) as obtained in Example 1-A) in methanol (30 ml), followed by 16 hours' stirring under reflux. The reaction mixture was concentrated and the residue was dissolved in ether. The solution was washed with 1N hydrochloric acid, a saturated aqueous solution of sodium bicarbonate and saturated brine in order, and dried over magnesium sulfate, followed by concentration. The residue was purified by silica gel column chromatography (eluate : hexane-ethyl actate) to give 1.10 g of (2S)-1-(t-butoxycarbonyl)-2-(1-hydroxy-2-phenoxyethyl) pyrrolidine.

B) (2S)-1-(N-Benzyloxycarbonyl-L-prolyl)-2-(1-hydroxy-2-phenoxyethyl)pyrrolidine

4N Hydrochloric acid-dioxane (16 ml) was added to (2S)-1-(t-butoxycarbonyl)-2-(1-hydroxy-2-phenoxyethyl)pyrrolidine (1.10 g), and the mixture was stirred at room temperature for 1 hour. The reaction mixture was concentrated to dryness and the residue was dissolved in DMF (10 ml), to which were added known N-benzyloxycarbonyl-L-proline (850 mg), N-methylmorpholine (0.38 ml) and HOBt (0.68 g). The reaction mixture was cooled to -25°C, added with DCC (0.72 g), and stirred at a temperature between -25°C and 0°C for 3 hours and then at room temperature for 12 hours. The resultant dicyclohexylurea was filtered off and the filtrate was concentrated. The residue was dissolved in ethyl acetate, washed with a saturated aqueous solution of sodium bicarbonate and saturated brine in order, and dried over magnesium sulfate, followed by concentration. The residue was purified by silica gel column chromatography (eluate : chloroform-methanol) to give 340 mg of (2S)-1-(N-benzyloxycarbonyl-L-prolyl)-2-(1-hydroxy-2-phenoxyethyl)pyrrolidine.

C) (2S)-1-(N-Benzyloxycarbonyl-L-prolyl)-2-(phenoxyacetyl)pyrrolidine

(2S)-1-(N-Benzyloxycarbonyl-L-prolyl)-2-(1-hydroxy-2-phenoxyethyl)pyrrolidine (393 mg) was subjected to DMSO-DCC oxidation in the same manner as in Example 1-D) to give 328 mg of (2S)-1-(N-benzyloxycarbonyl-L-prolyl)-2-(phenoxyacetyl)pyrrolidine(See Table 1).

Example 5

(2S)-1-{N-[(2-Oxo-1-pyrrolidinyl)acetyl]-L-prolyl}-2-[(phenylsulfinyl)acetyl]pyrrolidine (Compound 5)

A) N-[(2-Oxo-1-pyrrolidinyl)acetyl]-L-proline methyl ester

N-Methylmorpholine (5.50 ml), (2-oxo-1-pyrrolidinyl)acetic acid (7.15 g) and HOBt (10.12 g) were added to L-proline methyl ester hydrochloride (8.28 g) in DMF (100 ml) under ice-cooling. The mixture was cooled to -25°C, added with DCC (10.32 g) and stirred at room temperature for 16 hours. The resultant dicyclohexylurea was filtered off and the filtrate was concentrated. The residue was purified by silica gel column chromatography (eluate : chloroform-methanol) to give 12.94 g of N-[(2-oxo-1-pyrrolidinyl)acetyl]-L-proline methyl ester.

B) N-[(2-Oxo-1-pyrrolidinyl)acetyl]-L-proline

2N Sodium hydroxide solution (12 ml) was dropwise added to N-[(2-oxo-1-pyrrolidinyl)acetyl]-L-proline methyl ester (5.06 g) in methanol (40 ml) under ice-cooling, and the mixture was stirred at room temperature for 3 hours. 3N Hydrochloric acid was added to the reaction mixture under ice-cooling to adjust the pH to 5. The reaction mixture was concentrated to dryness, suspended in chloroform and filtered. The filtrate was concentrated to give 4.28 g of N-[(2-oxo-1-pyrrolidinyl)acetyl]-L-proline.

C) (2S)-2-[1-Hydroxy-2-(phenylthio)ethyl]-1-{N-[(2-oxo-1-pyrrolidinyl)acetyl]-L-prolyl}pyrrolidine

4N Hydrochloric acid-dioxane (40 ml) was added to (2S)-1-(t-butoxycarbonyl)-2-[1-hydroxy-2-(phenylthio)ethyl]pyrrolidine (2.62 g) as obtained in Example 1-B), and the mixture was stirred at room temperature for 1 hour. The reaction mixture was concentrated to dryness, and the residue was dissolved in DMF (20 ml), to which N-[(2-oxo-1-pyrrolidinyl)acetyl]-L-proline (1.95 g), N-methylmorpholine (0.9 ml) and HOBt (1.31 g) were added. The reaction mixture was cooled to -30°C, added with DCC (1.68 g) and stirred at a temperature between -30°C and 0°C for 3 hours, and then at room temperature for 16 hours. The reaction mixture was treated and purified in the same manner as in Example 1-C) to give 2.95 g of (2S)-2-[1-hydroxy-2-(phenylthio)ethyl]-1-{N-[(2-oxo-1-pyrrolidinyl)acetyl]-L-prolyl}pyrrolidine.

D) (2S)-2-[1-Hydroxy-2-(phenylsulfinyl)ethyl]-1-{N-[(2-oxo-1-pyrrolidinyl)acetyl]-L-prolyl}pyrrolidine

m-Chloroperbenzoic acid (1.09 g) was added to (2S)-2-[1-hydroxy-2-(phenylthio)ethyl]-1-{N-[(2-oxo-1-pyrrolidinyl)acetyl]-L-prolyl)pyrrolidine (2.81 g) in dichloromethane (15 ml) under ice-cooling, followed by stirring for 2 hours. The reaction mixture was treated in the same manner as in Example 2-A) and the residue was purified by silica gel column chromatography to give 2.63 g of (2S)-2-[1-hydroxy-2-(phenylsulfinyl)ethyl]-1-{N-[(2-oxo-1-pyrrolidinyl)acetyl]-L-prolyl}pyrrolidine.

E) (2S)-1-{N-[(2-Oxo-1-pyrrolidinyl)acetyl]-L-prolyl}-2-[(phenylsulfinyl)acetyl]pyrrolidine.

(2S)-2-[1-Hydroxy-2-(phenylsulfinyl)ethyl]-1-{N-[(2-oxo-1-pyrrolidinyl)acetyl]-L-prolyl}pyrrolidine (938 mg) was subjected to DMSO-DCC oxidation in the same manner as in Example 1-D) to give 817 mg of (2S)-1-{N-[(2-oxo-1-pyrrolidinyl)acetyl]-L-prolyl}-2-[(phenylsulfinyl)acetyl]pyrrolidine (See Table 2).

Example 6

(2S)-1-[4-(4-Chlorobenzylamino)-4-oxobutyryl]-2-[(phenylsulfinyl)acetyl]pyrrolidine (Compound 6)

A) (2S)-1-[4-(4-Chlorobenzylamino)-4-oxobutyryl]-2-[1-hydroxy-2-(phenylthio)ethyl]pyrrolidine

4N Hydrochloric acid-dioxane (25 ml) was added to (2S)-1-(t-butoxycarbonyl)-2-[1-hydroxy-2-(phenylthio)ethyl]pyrrolidine (3.28 g) as obtained in Example 1-B), and the mixture was stirred at room

temperature for 1 hour. The reaction mixture was concentrated to dryness, and the residue was dissolved in DMF (20 ml), to which known 4-(4-chlorobenzylamino)-4-oxobutyric acid (2.46 g), N-methylmorpholine (1.2 ml) and HOBt (1.64 g) were added. The mixture was cooled to -25° C, added with DCC (2.09 g) and stirred at a temperature between -25° C and 0° C for 3 hours, and at room temperature for 16 hours. The reaction mixture was treated and purified in the same manner as in Example 1-C) to give 3.75 g of (2S)-1-[4-(4-chlorobenzylamino)-4-oxobutyryl]-2-[1-hydroxy-2-(phenylthio)ethyl]pyrrolidine.

B) (2S)-1-[4-(4-Chlorobenzylamino)-4-oxobutyryl]-2-[1-hydroxy-2-(phenylsulfinyl)ethyl]pyrrolidine

(2S)-1-[4-(4-Chlorobenzylamino)-4-oxobutyryl]-2-[1-hydroxy-2-(phenylthio)ethyl]pyrrolidine (3.75 g) was subjected to oxidation in the same manner as in Example 2-A) in dichloromethane with 1.1 equivalent of m-chloroperbenzoic acid to give 3.70 g of (2S)-1-[4-(4-chlorobenzylamino)-4-oxobutyryl]-2-[1-hydroxy-2-(phenylsulfinyl)ethyl]pyrrolidine.

C) (2S)-1-[4-(4-Chlorobenzylamino)-4-oxobutyryl]-2-[(phenylsulfinyl)acetyl]pyrrolidine

(2S)-1-[4-(4-Chlorobenzylamino)-4-oxobutyryl]-2-[1-hydroxy-2-(phenylsulfinyl)ethyl]pyrrolidine (1.09 g) was subjected to DMSO-DCC oxidation as in Example 1-D) to give 974 mg of (2S)-1-[4-(4-chlorobenzylamino)-4-oxobutyryl]-2-[(phenylsulfinyl)acetyl]pyrrolidine (See Table 2).

## Example 7

(2S)-1-[N-(4-Phenylbutyryl)-L-prolyl]-2-[(phenylsulfinyl)acetyl]pyrrolidine (Compound 7)

A) N-(4-Phenylbutyryl)-L-proline benzyl ester

N-Methylmorpholine (3.4 ml) and 4-phenylbutyric acid (5.02 g) were added to L-proline benzyl ester hydrochloride (7.37 g) in dichloromethane (80 ml) under ice-cooling. The mixture was cooled to -25° C, added with DCC (6.31 g) and stirred at room temperature for 16 hours. The reaction mixture was treated and purified in the same manner as in Example 5, A) to give 9.42 g of N-(4-phenylbutyryl)-L-proline benzyl ester.

B) N-(4-phenylbutyryl)-L-proline

To N-(4-phenylbutyryl)-L-proline benzyl ester (9.31 g) in 99% methanol (100 ml) was added 10% Pd-C (0.90 g), and the mixture was stirred at room temperature in hydrogen stream for 2 hours. The catalyst was filtered off with celite, and the filtrate was concentrated to give 6.26 g of N-(4-phenylbutyryl)-L-proline.

C) (2S)-2-[1-Hydroxy-2-(phenylthio)ethyl]-1-[N-(4-phenylbutyryl)-L-prolyl]pyrrolidine

4N Hydrochloric acid-dioxane (23 ml) was added to (2S)-1-(t-butoxycarbonyl)-2-[1-hydroxy-2-(phenylthio)ethyl]pyrrolidine (3.05 g) as obtained in Example 1-B), and the mixture was stirred at room temperature for 0.5 hour. The reaction mixture was concentrated to dryness, and the residue was dissolved in DMF (18 ml), to which N-(4-phenylbutyryl)-L-proline (2.65 g), N-methylmorpholine (1.2 ml) and HOBt (1.88 g) were added. The mixture was cooled to -25° C, added with DCC (1.95 g) and stirred at a temperature between -25° C and 0° C for 2 hours and then at room temperature for 14 hours. The reaction mixture was treated and purified in the same manner as in Example 1-C) to give 2.45 g of (2S)-2-[1-hydroxy-2-(phenylthio)ethyl]-1-[N-(4-phenylbutyryl)-L-prolyl]pyrrolidine.

D) (2S)-2-[1-Hydroxy-2-(phenylsulfinyl)ethyl]-1-[N-(4-phenylbutyryl)-L-prolyl]pyrrolidine

(2S)-2-[1-Hydroxy-2-(phenylthio)ethyl]-1-[N-(4-phenylbutyryl)-L-prolyl]pyrrolidine (1.25 g) was subjected to oxidation in the same manner as in Example 2-A) in dichloromethane (9 ml) using 1.1 equivalent of m-chloroperbenzoic acid to give 1.18 g of (2S)-2-[1-hydroxy-2-(phenylsulfinyl)ethyl]-1-[N-(4-phenylbutyryl)-L-prolyl]pyrrolidine.

E) (2S)-1-[N-(4-Phenylbutyryl)-L-prolyl]-2-[(phenylsulfinyl)acetyl]pyrrolidine

(2S)-2-[1-Hydroxy-2-(phenylsulfinyl)ethyl]-1-[N-(4-phenylbutyryl)-L-prolyl]pyrrolidine (1.07 g) was subjected to DMSO-DCC oxidation as in Example 1-D) to give 0.89 g of (2S)-1-[N-(4-phenylbutyryl)-L-prolyl]-2-[(phenylsulfinyl)acetyl]pyrrolidine (See Table 8).

Example 8

(2S)-1-[N-(Benzyloxycarbonyl)glycyl]-2-[(phenylsulfinyl)acetyl]pyrrolidine (Compound 8)

A) (2S)-1-[N-(Benzyloxycarbonyl)glycyl]-2-[1-hydroxy-2-(phenylthio)ethyl]pyrrolidine

4N Hydrochloric acid-dioxane (20 ml) was added to (2S)-1-(t-butoxycarbonyl)-2-[1-hydroxy-2-(phenylthio)ethyl]pyrrolidine (2.65 g) as obtained in Example 1-B), and the mixture was stirred at room temperature for 0.5 hour. The reaction mixture was concentrated to dryness, and the residue was dissolved in DMF (15 ml), to which known N-(benzyloxycarbonyl)glycine (1.71 g), N-methylmorpholine (0.9 ml) and HOBt (1.33 g) were added. The mixture was cooled to -25° C, added with DCC (1.70 g) and stirred at a temperature between -25° C and 0° C for 2 hours and then at room temperature for 14 hours. The reaction mixture was treated and purified in the same manner as in Example 1-C) to give 3.06 g of (2S)-1-[N-(benzyloxycarbonyl)glycyl]-2-[1-hydroxy-2-(phenylthio)ethyl]pyrrolidine.

B) (2S)-1-[N-(Benzyloxycarbonyl)glycyl]-2-[1-hydroxy-2-(phenylsulfinyl)ethyl]pyrrolidine

(2S)-1-[N-(Benzyloxycarbonyl)glycyl]-2-[1-hydroxy-2-(phenylthio)ethyl]pyrrolidine (1.32 g) was subjected to oxidation in the same manner as in Example 2-A) in dichloromethane (10 ml) using 1.1 equivalent of m-chloroperbenzoic acid to give 1.35 g of (2S)-1-[N-(benzyloxycarbonyl)glycyl]-2-[1-hydroxy-2-(phenylsulfinyl)-ethyl]pyrrolidine.

C) (2S)-1-[N-(Benzyloxycarbonyl)glycyl]-2-[(phenylsulfinyl)acetyl]pyrrolidine

(2S)-1-[N-(Benzyloxycarbonyl)glycyl]-2-[1-hydroxy-2-(phenylsulfinyl)ethyl]pyrrolidine (1.30 g) was subjected to DMSO-DCC oxidation as in Example 1-D) to give 0.94 g of (2S)-1-[N-(benzyloxycarbonyl)glycyl]-2-[(phenylsulfinyl)acetyl]pyrrolidine (See Table 2).

Example 9

(2S)-1-(N-Benzyloxycarbonyl-L-prolyl)-2-[(4-methoxyphenylsulfinyl)acetyl]pyrrolidine (Compound 9)

A) (2S)-1-(t-Butoxycarbonyl)-2-[1-hydroxy-2-(4-methoxyphenylthio)ethyl]pyrrolidine

4-Methoxythiophenol (1.0 ml) and triethylamine (1.0 ml) were added to (2S)-1-(t-butoxycarbonyl)-2-(1,2-epoxyethyl)pyrrolidine (1.57 g) as obtained in Example 1-A) in methanol (50 ml), and the mixture was stirred for 1.5 hours under reflux. The reaction mixture was concentrated and the residue was poured into ice-water, followed by extraction with ethyl acetate. The extract was washed with saturated brine, dried over magnesium sulfate and concentrated. The residue was recrystallized from a mixted solution of hexane-ethyl acetate to give 2.16 g of the title compound as colorless needles.

B) (2S)-1-(N-Benzyloxycarbonyl-L-prolyl)-2-[1-hydroxy-2-(4-methoxyphenylthio)ethyl]pyrrolidine

4N Hydrochloric acid-dioxane (15 ml) was added to (2S)-1-(t-butoxycarbonyl)-2-[1-hydroxy-2-(4-methoxyphenylthio)ethyl]pyrrolidine (2.02 g), and the mixture was stirred at room temperature for 0.5 hour. The reaction mixture was concentrated to dryness, and the residue was dissolved in DMF (15 ml), followed by condensation reaction as in Example 1-C) with known N-benzyloxycarbonyl-L-proline (1.42 g) to give 2.51 g of the title compound.

C) (2S)-1-(N-Benzyloxycarbonyl-L-prolyl)-2-[1-hydroxy-2-(4-methoxyphenylsulfinyl)ethyl]pyrrolidine

(2S)-1-(N-Benzyloxycarbonyl-L-prolyl)-2-[1-hydroxy-2-(4-methoxyphenylthio)ethyl]pyrrolidine (1.51 g) was subjected to oxidation as in Example 2-A) in dichloromethane using 1.1 equivalent of m-chloroperbenzoic acid to give 984 mg of the title compound.

D) (2S)-1-(N-Benzyloxycarbonyl-L-prolyl)-2-[(4-methoxyphenylsulfinyl)acetyl]pyrrolidine

(2S)-1-(N-Benzyloxycarbonyl-L-prolyl)-2-[1-hydroxy-2-(4-methoxyphenylsulfinyl)ethyl]pyrrolidine (901 mg) was subjected to DMSO-DCC oxidation as in Example 1-D) to give 652 mg of the title compound (See Table 3).

Example 10

(2S)-1-(N-Benzyloxycarbonyl-L-prolyl)-2-[(4-nitrophenylsulfinyl)acetyl]pyrrolidine (Compound 10)

A) (2S)-1-(t-Butoxycarbonyl)-2-[1-hydroxy-2-(4-nitrophenylthio)ethyl]pyrrolidine

4-Nitrothiophenol (1.21 g) and triethylamine (0.91 ml) were added to (2S)-1-(t-butoxycarbonyl)-2-(1,2-epoxyethyl)pyrrolidine (1.39 g) as obtained in Example 1-A) in methanol (50 ml), and the mixture was stirred for 2 hours under reflux. The reaction mixture was concentrated and the residue was purified by silica gel column chromatography (eluate : hexane-ethyl acetate) to give 1.85 g of the title compound.

B) (2S)-1-(N-Benzyloxycarbonyl-L-prolyl)-2-[1-hydroxy-2-(4-nitrophenylthio)ethyl]pyrrolidine

4N Hydrochloric acid-dioxane (20 ml) was added to (2S)-1-(t-butoxycarbonyl)-2-[1-hydroxy-2-(4-nitrophenylthio)ethyl]pyrrolidine (1.64 g), and the mixture was stirred at room temperature for 0.5 hour. The reaction mixture was concentrated to dryness, and the residue was dissolved in DMF (12 ml), followed by condensation reaction as in Example 1-C) with known N-benzyloxycarbonyl-L-proline (1.10 g) to give 2.03 g of the title compound.

C) (2S)-1-(N-Benzyloxycarbonyl-L-prolyl)-2-[1-hydroxy-2-(4-nitrophenylsulfinyl)ethyl]pyrrolidine

(2S)-1-(N-Benzyloxycarbonyl-L-prolyl)-2-[1-hydroxy-2-(4-nitrophenylthio)ethyl]pyrrolidine (1.50 g) was subjected to oxidation as in Example 2-A) in dichloromethane using 1.1 equivalent of m-chloroperbenzoic acid to give 894 mg of the title compound.

D) (2S)-1-(N-Benzyloxycarbonyl-L-prolyl)-2-[(4-nitrophenylsulfinyl)acetyl]pyrrolidine

(2S)-1-(N-Benzyloxycarbonyl-L-prolyl)-2-[1-hydroxy-2-(4-nitrophenylsulfinyl)ethyl]pyrrolidine (883 mg) was subjected to DMSO-DCC oxidation as in Example 1-D) to give 673 mg of the title compound (See Table 3).

Example 11

(2S)-1-(N-Benzyloxycarbonyl-L-prolyl)-2-[(isopropylsulfinyl)acetyl]pyrrolidine (Compound 11)

A) (2S)-1-(t-Butoxycarbonyl)-2-[1-hydroxy-2-(isopropylthio)ethyl]pyrrolidine

Isopropylmercaptan (0.93 ml) and triethylamine (1.39 ml) were added to (2S)-1-(t-butoxycarbonyl)-2-(1,2-epoxyethyl)pyrrolidine (2.11 g) as obtained in Example 1-A) in methanol (100 ml), and the mixture was stirred for 2 hours under reflux. The reaction mixture was concentrated and the residue was purified by silica gel column chromatography (eluate : hexane-ethyl acetate) to give 1.66 g of the title compound.

B) (2S)-1-(N-Benzyloxycarbonyl-L-prolyl)-2-[1-hydroxy-2-(isopropylthio)ethyl]pyrrolidine

4N Hydrochloric acid-dioxane (25 ml) was added to (2S)-1-(t-butoxycarbonyl)-2-[1-hydroxy-2-(isopropylthio)ethyl]pyrrolidine (1.68 g), and the mixture was stirred at room temperature for 0.5 hour. The reaction mixture was concentrated to dryness, and the residue was dissolved in DMF (12 ml), followed by the condensation reaction as in Example 1-C) with known N-benzyloxycarbonyl-L-proline (1.46 g) to give 2.08 g of the title compound.

C) (2S)-1-(N-Benzyloxycarbonyl-L-prolyl)-2-[1-hydroxy-2-(isopropylsulfinyl)ethyl]pyrrolidine

(2S)-1-(N-Benzyloxycarbonyl-L-prolyl)-2-[1-hydroxy-2-(isopropylthio)ethyl]pyrrolidine (1.61 g) was subjected to oxidation as in Example 2-A) in dichloromethane using 1.1 equivalent of m-chloroperbenzoic acid to give 1.40 g of the title compound.

D) (2S)-1-(N-Benzyloxycarbonyl-L-prolyl)-2-[(isopropylsulfinyl)acetyl]pyrrolidine

(2S)-1-(N-Benzyloxycarbonyl-L-prolyl)-2-[1-hydroxy-2-(isopropylsulfinyl)ethyl]pyrrolidine (1.34 g) was subjected to DMSO-DCC oxidation as in Example 1-D) to give 502 mg of the title compound (See Table 3).

Example 12

(2S)-1-(N-Benzyloxycarbonyl-L-prolyl)-2-{[(S)-phenylsulfinyl]acetyl}pyrrolidine (Compound 12)

A) (2S)-1-(N-Benzyloxycarbonyl-L-prolyl)-2-{1-hydroxy-2-[(S)-phenylsulfinyl]ethyl}pyrrolidine

(2S)-1-(N-Benzyloxycarbonyl-L-prolyl)-2-[1-hydroxy-2-(phenylsulfinyl)ethyl]pyrrolidine (6.72 g) as obtained in Example 2-A) was again purified by medium pressure liquid chromatography (silica gel, eluate ; chloroform:methanol = 98:2) to give the title compound (2.51 g), the corresponding (R)-phenyl-sulfinyl compound (2.20 g) and a mixture of the two (1.87 g).

B) (2S)-1-(N-Benzyloxycarbonyl-L-prolyl)-2-{[(S)-phenylsulfinyl]acetyl}pyrrolidine

(2S)-1-(N-Benzyloxycarbonyl-L-prolyl)-2-{1-hydroxy-2-[(S)-phenylsulfinyl]ethyl}pyrrolidine (600 mg) was subjected to DMSO-DCC oxidation as in Example 1-D), followed by purification of the reaction product by medium pressure liquid chromatography (silica gel, eluate; chloroform:methanol = 99.5:0.5) to give 242 mg of the title compound (See Table 3).

Example 13

(2S)-1-(N-Benzyloxycarbonyl-L-prolyl)-2-{[(R)-phenylsulfinyl]acetyl}pyrrolidine (Compound 13)

(2S)-1-(N-Benzyloxycarbonyl-L-prolyl)-2-{1-hydroxy-2-[(R)-phenylsulfinyl]ethyl}pyrrolidine (1.00 g) as obtained in Example 12-A) was subjected to DMSO-DCC oxidation as in Example 1-D), followed by purification of the reaction product by medium pressure liquid chromatography (silica gel, eluate ; chloroform:methanol = 99.5:0.5). The oily substance (500 mg) was recrystallized from acetone-ether-hexane to give 300 mg of the title compound as colorless needles (See Table 4).

Example 14

(2S)-1-(N-Benzyloxycarbonyl-L-prolyl)-2-{[(S)-4-methoxyphenylsulfinyl]acetyl}pyrrolidine (Compound 14)

A) (2S)-1-(N-Benzyloxycarbonyl-L-prolyl)-2-{1-hydroxy-2-[(S)-4-methoxyphenylsulfinyl]ethyl}pyrrolidine

(2S)-1-(N-Benzyloxycarbonyl-L-prolyl)-2-[1-hydroxy-2-(4-methoxyphenylsulfinyl)ethyl]pyrrolidine (575 mg) as obtained in Example 9-C) was again purified by medium pressure liquid chromatography (silica gel, eluate ; chloroform:methanol = 98.5:1.5) to give the title compound (164 mg), the corresponding (R)-4-methoxyphenylsulfinyl compound (221 mg) and a mixture of the two (175 mg).

B) (2S)-1-(N-Benzyloxycarbonyl-L-prolyl)-2-{[(S)-4-methoxyphenylsulfinyl]acetyl}pyrrolidine

(2S)-1-(N-Benzyloxycarbonyl-L-prolyl)-2-{1-hydroxy-2-[(S)-4-methoxyphenylsulfinyl]ethyl}pyrrolidine (152 mg) was subjected to DMSO-DCC oxidation as in Example 1-D), and the reaction product was purified by silica gel preparative thin-layer chromatography (developing solvent ; chloroform:methanol = 98:2, double development) to give 97 mg of the title compound (See Table 4).

Example 15

(2S)-1-(N-Benzyloxycarbonyl-L-prolyl)-2-{[(R)-4-methoxyphenylsulfinyl]acetyl}pyrrolidine (Compound 15)

(2S)-1-(N-Benzyloxycarbonyl-L-prolyl)-2-{1-hydroxy-2-[(R)-4-methoxyphenylsulfinyl]ethyl}pyrrolidine (182 mg) as obtained in Example 14-A) was subjected to DMSO-DCC oxidation as in Example 1-D), and the reaction product was purified by silica gel preparative thin-layer chromatography (developing solvent ; chloroform:methanol = 99:1, triple development) to give 134 mg of the title compound (See Table 4).

Example 16

(2S)-1-(N-Benzyloxycarbonyl-L-prolyl)-2-{[(S)-p-tolylsulfinyl]acetyl}pyrrolidine (Compound 16)

A) (2S)-1-(N-Benzyloxycarbonyl-L-prolyl)-2-{1-hydroxy-2-[(S)-p-tolylsulfinyl]ethyl}pyrrolidine

To diisopropylamine (0.26 ml) in THF (6 ml) was dropwise added 1.62 M n-butyllithium-hexane solution (1.12 ml) at -78 °C, and the mixture was stirred at 0 °C for 0.5 hour. The reaction mixture was cooled to -30 °C, dropwise added with (S)-(-)-methyl p-tolyl sulfoxide (255 mg) in THF (6 ml), and stirred at 0 °C for 0.5 hour. The reaction mixture was cooled to -78 °C and dropwise added with known N-benzyloxycarbonyl-L-prolyl-L-prolinal (300 mg) in THF (6 ml). After stirring for 0.5 hour, an aqueous solution of saturated ammonium chloride was added thereto, followed by stirring at room temperature for 10 minutes. The reaction mixture was extracted with ethyl acetate, and the organic layer was washed with a saturated aqueous solution of sodium bicarbonate and saturated brine in order, dried over magnesium sulfate and concentrated. The residue was purified by silica gel column chromatography (eluate ; chloroform-methanol) to give 388 mg of the title compound.

B) (2S)-1-(N-Benzyloxycarbonyl-L-prolyl)-2-{[(S)-p-tolylsulfinyl]acetyl}pyrrolidine

(2S)-1-(N-Benzyloxycarbonyl-L-prolyl)-2-{1-hydroxy-2-[(S)-p-tolylsulfinyl]ethyl}pyrrolidine (357 mg) was subjected to DMSO-DCC oxidation as in Example 1-D), and the reaction product was purified by medium pressure liquid chromatography (silica gel, eluate ; chloroform:methanol = 99:1) to give 162 mg of the title compound (See Table 4).

Example 17

(2S)-1-(N-Benzyloxycarbonyl-L-prolyl)-2-{[(R)-p-tolylsulfinyl]acetyl}pyrrolidine (Example 17)

By the same procedure as in Example 16 using (R)-(+)-methyl p-tolyl sulfoxide (43 mg) in place of (S)-(-)-methyl p-tolyl sulfoxide, 32 mg of the title compound was obtained (See Table 5).

Example 18

(2S)-2-{[(S)-4-Methoxyphenylsulfinyl]acetyl}-1-[N-(3-phenylpropionyl)-L-prolyl]pyrrolidine (Compound 18)

A) N-(3-phenylpropionyl)-L-proline

By the same procedure as in Example 7-A) and B) using 3-phenylpropionic acid (13.67 g) in place of 4-phenylbutyric acid, 17.36 g of the title compound was obtained.

B) N-(3-Phenylpropionyl)-L-prolyl-L-proline benzyl ester

To L-proline benzyl ester hydrochloride (0.98 g) in dichloromethane (20 ml) were added N-methylmorpholine (0.49 ml), N-(3-phenylpropionyl)-L-proline (1.00 g) and HOBt (0.60 g) under ice-cooling. After cooling to -25 °C, DCC (0.92 g)was added, and the mixture was stirred at a temperature between -25 °C and 0 °C for 2 hours, and then at room temperature for 14 hours. The reaction mixture was treated and purified as in Example 5-A) to give 1.39 g of the title compound.

C) (S)-(-)-4-Methoxyphenyl methyl sulfoxide

To titanium tetraisopropoxide (1.49 ml) in dichloromethane (50 ml) were added diethyl (S,S)-tartrate (1.71 ml) and water (90 µl), and the mixture was stirred at room temperature for 20 minutes. After 1-methoxy-4-(methylthio)benzene (0.69 ml) was dropwise added, the reaction mixture was cooled to -20 °C,

dropwise added with 3.07 M t-butylhydroperoxide-toluene solution (1.8 ml), and stirred at -20° C for 15 hours. Water (0.9 ml) was added, and the mixture was stirred at -20° C for 1 hour, and then at room temperature for 1 hour. A small amount of alumina was added, and the reaction mixture was suction-filtered with celite and washed well with dichloromethane. The filtrate and the washing were combined, added with 5% sodium hydroxide-saturated brine and stirred at room temperature for 1 hour. The organic layer was separated, dried over magnesium sulfate and concentrated. The residue was purified by silica gel column chromatography (eluate; ethyl acetate), and the residue (730 mg) was recrystallized from ether-pentane to give 586 mg of the title compound, $[\alpha]_D$ -103° (c = 1.98, acetone).

D) (2S)-2-{[(S)-4-methoxyphenylsulfinyl]acetyl}-1-[N-(3 phenylpropionyl)-L-prolyl]pyrrolidine

To diisopropylamine (0.13 ml) in THF (8 ml) was dropwise added 1.62 M n-butyllithium-hexane solution (1.2 ml) at -78° C, followed by stirring at 0° C for 0.5 hour. The reaction mixture was cooled to -30° C, dropwise added with (S)-(-)-4-methoxyphenyl methyl sulfoxide (157 mg) in THF (8 ml), and stirred at 0° C for 0.5 hour. The reaction mixture was cooled to -78° C, added with N-(3-phenylpropionyl)-L-prolyl-L-proline benzyl ester (400 mg) in THF (8 ml) quickly, and stirred for 0.5 hour. The reaction mixture was treated as in Example 16-A) and the residue obtained was purified by medium pressure liquid chromatography (silica gel, eluate ; chloroform:methanol = 99:1) to give 195 mg of the title compound (See Table 5).

Example 19

(2S)-1-[N-(3-Phenylpropionyl)-L-prolyl]-2-{[(S)-p-tolylsulfinyl]acetyl}pyrrolidine (Compound 19)

The oily substance (231 mg) obtained by the same procedure as in Example 18-D) using (S)-(-)-methyl p-tolyl sulfoxide (142 mg) in place of (S)-(-)-4-methoxyphenyl methyl sulfoxide was recrystallized from hexane-ethyl acetate to give 160 mg of the title compound as colorless needles (See Table 5).

Example 20

(2S)-1-[N-(3-Phenylpropionyl)-L-prolyl]-2-{[(S)-phenylsulfinyl]acetyl}pyrrolidine (Compound 20)

A) (S)-(-)-methyl phenyl sulfoxide

By the same procedure as in Example 18-C) using thioanisole (5.87 ml) in place of 1-methoxy-4-(methylthio)benzene, 4.63 g of the title compound was obtained, $[\alpha]_D$ -125° (c = 1.81, acetone).

B) (2S)-1-[N-(3-phenylpropionyl)-L-prolyl]-2-{[(S)-phenylsulfinyl]acetyl}pyrrolidine

The crystalline substance (202 mg) as obtained by the same procedure as in Example 18-D) using (S)-(-)-methyl phenyl sulfoxide (129 mg) in place of (S)-(-)-4-methoxyphenyl methyl sulfoxide was recrystallized from hexane-ethyl acetate to give 155 mg of the title compound as colorless needles (See Table 5).

Example 21

(2S)-1-(N-Benzyloxycarbonyl-L-prolyl)-2-[(4-methoxyphenoxy)acetyl]pyrrolidine (Compound 21)

A) (2S)-1-[t-Butoxycarbonyl)-2-[1-hydroxy-2-(4-methoxyphenoxy)ethyl]pyrrolidine

To (2S)-1-[t-butoxycarbonyl)-2-(1,2-epoxyethyl)pyrrolidine (11.70 g) as obtained in Example 1-A) in methanol (50 ml) were added 4-methoxyphenol (13.60 g) and 1 M sodium methoxide-methanol solution (54.9 ml), and the mixture was stirred at 70° C for 18 hours. The reaction mixture was poured into a satuated aqueous solution of ammonium chloride and extracted with ethyl acetate. The extract was washed with saturated brine, dried over magnesium sulfate and concentrated. The residue was purified by silica gel column chromatography (eluate; hexane-ethyl acetate) to give 14.20 g of the title compound.

B) (2S)-1-(N-Benzyloxycarbonyl-L-prolyl)-2-[1-hydroxy-2-(4-methoxyphenoxy)ethyl]pyrrolidine

To (2S)-1-[t-butoxycarbonyl)-2-[1-hydroxy-2-(4-methoxyphenoxy)ethyl]pyrrolidine (14.03 g) was added

4N hydrochloric acid-dioxane (220 ml), and the mixture was stirred at room temperature for 1 hour. The reaction mixture was concentrated to dryness, and the residue was dissolved in DMF (100 ml), followed by condensation reaction as in Example 1-C) with known N-benzyloxycarbonyl-L-proline (10.70 g) to give 11.10 g of the title compound.

C) (2S)-1-(N-Benzyloxycarbonyl-L-prolyl)-2-[(4-methoxyphenoxy)acetyl]pyrrolidine

(2S)-1-(N-Benzyloxycarbonyl-L-prolyl)-2-[1-hydroxy-2-(4-methoxyphenoxy)ethyl]pyrrolidine (10.94 g) was subjected to DMSO-DCC oxidation in the same manner as in Example 1-D) to give 10.78 g of the title compound (See Table 6).

Example 22

(2S)-2-(Phenoxyacetyl)-1-[N-(3-phenylpropionyl)-L-prolyl]pyrrolidine (Compound 22)

A) (2S)-2-(1-hydroxy-2-phenoxyethyl)-1-[N-(3-phenylpropionyl)-L-prolyl]pyrrolidine

(2S)-1-(t-Butoxycarbonyl)-2-(1-hydroxy-2-phenoxyethyl)pyrrolidine (1.20 g) as obtained in Example 4-A) was dissolved in 4N hydrochloric acid-dioxane (20 ml), and the mixture was stirred at room temperature for 0.5 hour. The reaction mixture was concentrated to dryness, and the residue was dissolved in DMF (9 ml), followed by condensation reaction as in Example 1-C) with N-(3-phenylpropionyl)-L-proline (0.96 g) as obtained in Example 18-A) to give 461 mg of the title compound.

B) (2S)-2-(Phenoxyacetyl)-1-[N-(3-phenylpropionyl)-L-prolyl]pyrrolidine

(2S)-2-(1-Hydroxy-2-phenoxyethyl)-1-[N-(3-phenylpropionyl)L-prolyl]pyrrolidine (425 mg) was subjected to DMSO-DCC oxidation as in Example 1-D) to give 152 mg of the title compound (See Table 6).

Example 23

(2S)-2-[(4-Methoxyphenoxy)acetyl]-1-[N-(3-phenylpropionyl)-L-prolyl]pyrrolidine (Compound 23)

A) (2S)-2-[1-Hydroxy-2-(4-methoxyphenoxy)ethyl]-1-[N-(3-phenylpropionyl)-L-prolyl]pyrrolidine

(2S)-1-(t-Butoxycarbonyl)-2-[1-hydroxy-2-(4-methoxyphenoxy)ethyl]pyrrolidine (455 mg) as obtained in Example 21-A) was dissolved in 4N hydrochloric acid-dioxane (7 ml), and the mixture was stirred at room temperature for 0.5 hour. The reaction mixture was concentrated to dryness, and the residue was dissolved in DMF (7 ml), followed by condensation reaction as in Example 1-C) with N-(3-phenylpropionyl)-L-proline (335 mg) as obtained in Example 18-A) to give 138 mg of the title compound.

B) (2S)-2-[(4-Methoxyphenoxy)acetyl]-1-[N-(3-phenylpropionyl)-L-prolyl]pyrrolidine

(2S)-2-[1-Hydroxy-2-(4-methoxyphenoxy)ethyl]-1-[N-(3-phenylpropionyl)-L-prolyl]pyrrolidine (128 mg) was subjected to DMSO-DCC oxidation as in Example 1-D) to give 45 mg of the title compound (See Table 6).

Example 24

(2S)-1-(N-Benzylaminocarbonyl-L-prolyl)-2-(phenoxyacetyl)pyrrolidine (Compound 24)

A) (2S)-1-[N-(t-Butoxycarbonyl)-L-prolyl]-2-(1-hydroxy-2-phenoxyethyl)pyrrolidine

(2S)-1-(t-Butoxycarbonyl)-2-(1-hydroxy-2-phenoxyethyl)pyrrolidine (1.93 g) as obtained in Example 4-A) was dissolved in 4N hydrochloric acid-dioxane (31 ml), and the mixture was stirred at room temperature for 0.5 hour. The reaction mixture was concentrated to dryness, and the residue was dissolved in DMF (15 ml), followed by condensation reaction as in Example 1-C) with N-(t-butoxycarbonyl)-L-proline (1.36 g), N-methylmorpholine (0.69 ml), HOBt (1.02 g) and DCC (1.30 g) to give 2.10 g of the title compound.

B) (2S)-1-(N-Benzylaminocarbonyl-L-prolyl)-2-(1-hydroxy-2-phenoxyethyl)pyrrolidine

(2S)-1-[N-(t-Butoxycarbonyl)-L-prolyl]-2-(1-hydroxy-2-phenoxyethyl)pyrrolidine (0.99 g) was dissolved in 4N hydrochloric acid-dioxane (13 ml), and the mixture was stirred at room temperature for 0.5 hour. The reaction mixture was concentrated to dryness, and the residue was dissolved in dichloromethane (10 ml), to which N-methylmorpholine (0.27 ml) and benzylisocyanate (0.30 ml) were dropwise added in order under ice-cooling. After stirring for 1.5 hours, the reaction mixture was poured into ice-water and extracted with dichloromethane. The organic layer was washed with 1N hydrochloric acid, a saturated aqueous solution of sodium bicarbonate and saturated brine in order, dried over anhydrous magnesium sulfate, and concentrated. The residue was recrystallized from ethyl acetate-hexane to give 0.85 g of the title compound.

C) (2S)-1-(N-Benzylaminocarbonyl-L-prolyl)-2-(phenoxyacetyl)pyrrolidine

(2S)-1-(N-Benzylaminocarbonyl-L-prolyl)-2-(1-hydroxy-2-phenoxyethyl)pyrrolidine (798 mg) was subjected to DMSO-DCC oxidation as in Example 1-D), followed by recrystallization from ethyl acetate-hexane to give 427 mg of the title compound (See Table 6).

Example 25

(2S)-1-(N-Benzylaminocarbonyl-L-prolyl)-2-(4-methoxyphenoxyacetyl)pyrrolidine (Compound 25)

A) (2S)-1-[N-(t-Butoxycarbonyl)-L-prolyl]-2-[1-hydroxy-2-(4-methoxyphenoxy)ethyl]pyrrolidine

(2S)-1-(t-Butoxycarbonyl)-2-[1-hydroxy-2-(4-methoxyphenoxy)ethyl]pyrrolidine (1.57 g) as obtained in Example 21-A) was dissolved in 4N hydrochloric acid-dioxane (23 ml), and the mixture was stirred at room temperature for 1 hour. The reaction mixture was concentrated to dryness, and the residue was dissolved in DMF (15 ml), followed by condensation reaction as in Example 1-C) with N-(t-butoxycarbonyl)-L-proline (1.00 g), N-methylmorpholine (0.51 ml), HOBt (0.75 g) and DCC (0.96 g) to give 1.41 g of the title compound.

B) (2S)-1-(N-Benzylaminocarbonyl-L-prolyl)-2-[1-hydroxy-2-(4-methoxyphenoxy)ethyl]pyrrolidine

By the same procedure as in Example 24-B), (2S)-1-[N-(t-butoxycarbonyl)-L-prolyl]-2-[1-hydroxy-2-(4-methoxyphenoxy) ethyl]pyrrolidine (722 mg) was treated with 4N hydrochloric acid-dioxane), followed by reaction with benzylisocyanate (0.21 ml) to give 596 mg of the title compound as crystals.

C) (2S)-1-(N-Benzylaminocarbonyl-L-prolyl)-2-(4-methoxyphenoxyacetyl)pyrrolidine

(2S)-1-(N-Benzylaminocarbonyl-L-prolyl)-2-[1-hydroxy-2-(4-methoxyphenoxy)ethyl]pyrrolidine (557 mg) was dissolved in a mixed solution of DMSO (3 ml), benzene (1.5 ml) and triethylamine (0.6 ml) and added with sulfur trioxide-pyridine complex (0.52 g) under ice-cooling. After stirring at 5-10 °C for 1 hour, the reaction mixture was poured into ice-water and extracted with ethyl acetate. The organic layer was washed with 1N hydrochloric acid, a saturated aqueous solution of sodium bicarbonate and saturated brine in order, dried over anhydrous magnesium sulfate and concentrated. The residue was recrystallized from a mixture of ethyl acetate-hexane to give 374 mg of the title compound (See Table 7).

Example 26

(2S)-1-(N-Benzylaminocarbonyl-L-prolyl)-2-{[(S)-4-methoxyphenylsulfinyl]acetyl}pyrrolidine (Compound 26)

A) N-(t-Butoxycarbonyl)-L-prolyl-L-proline benzyl ester

To a suspension of L-proline benzyl ester hydrochloride (3.37 g) in dichloromethane (60 ml) were added N-methylmorpholine (1.54 ml), N-(t-butoxycarbonyl)-L-proline (3.00 g), HOBt (2.08 g) and water-soluble carbodiimide hydrochloride (2.95 g) in order, followed by stirring for 1 hour. After stirring at room temperature for 17 hours, the reaction mixture was poured into ethyl acetate, washed with 1N hydrochloric acid, a saturated aqueous solution of sodium bicarbonate and saturated brine in order, dried over anhydrous magnesium sulfate, and concentrated. The residue was purified by silica gel column chromatography

(eluate; hexane-ethyl acetate) to give 5.00 g of the title compound.

B) N-Benzylaminocarbonyl-L-prolyl-L-proline benzyl ester

N-(t-Butoxycarbonyl)-L-prolyl-L-proline benzyl ester (3.60 g) was treated with 4N hydrochloric acid-dioxane and then reacted with benzylisocyanate (1.2 ml) as in Example 24-B) to give 2.98 g of the title compound as crystals.

C) (2S)-1-(N-Benzylaminocarbonyl-L-prolyl)-2-{[(S)-4-methoxyphenylsulfinyl]acetyl}pyrrolidine

By the same procedure as in Example 18-D) using N-benzylaminocarbonyl-L-prolyl-L-proline benzyl ester (2.00 g) and (S)-(-)-4-methoxyphenyl methyl sulfoxide (780 mg) as obtained in Example 18-C), there was obtained 629 mg of the title compound as colorless needles (See Table 7).

Example 27

(2S)-1-[N-(4-Methoxybenzylaminocarbonyl)-L-prolyl]-2-{[(S)-4-methoxyphenylsulfinyl]acetyl}pyrrolidine (Compound 27)

A) N-(4-Methoxybenzylaminocarbonyl)-L-prolyl-L-proline benzyl ester

To trichloromethyl chloroformate (0.28 ml) in THF (40 ml) were dropwise added 4-methoxybenzylamine (0.61 ml) and triethylamine (0.65 ml) in THF (20 ml) at -20 °C over 10 minutes. After stirring for 1 hour, L-prolyl-L-proline benzyl ester hydrochloride (1.57 g) as obtained by treating N-(t-butoxycarbonyl)-L-prolyl-L-proline benzyl ester obtained in Example 26-A) with 4N hydrochloric acid-dioxane in dichloromethane (20 ml) and triethylamine (1.3 ml) were added dropwise thereto, followed by stirring for 0.5 hour. The reaction mixture was poured into ice-water and extracted with ethyl acetate. The organic layer was washed with 1N hydrochloric acid, a saturated aqueous solution of sodium bicarbonate and saturated brine in order, dried over anhydrous magnesium sulfate, and concentrated. The residue was purified by silica gel column chromatography (eluate; ethyl acetate) to give 1.34 g of the title compound.

B) (2S)-1-[N-(4-Methoxybenzylaminocarbonyl)-L-prolyl]-2-{[(S)-4-methoxyphenylsulfinyl]acetyl}pyrrolidine

By the same procedure as in Example 18-D) using N-(4-methoxybenzylaminocarbonyl)-L-prolyl-L-proline benzyl ester (1.30 g) and (S)-(-)-4-methoxyphenyl methyl sulfoxide (480 mg) as obtained in Example 18-C), there was obtained 460 mg of the title compound as colorless needles (See Table 7).

Example 28

(2S)-1-[N-(4-Methoxybenzylaminocarbonyl)-L-prolyl]-2-(4-methoxyphenoxyacetyl)pyrrolidine (Compound 28)

A) (2S)-2-[1-Hydroxy-2-(4-methoxyphenoxy)ethyl]-1-[N-(4-methoxybenzylaminocarbonyl)-L-prolyl]pyrrolidine

By the same procedure as in Example 27-A) using trichloromethyl chloroformate (0.20 ml), 4-methoxybenzylamine (449 mg), triethylamine (0.45 ml + 0.89 ml) and (2S)-2-[1-hydroxy-2-(4-methoxyphenoxy)ethyl]-1-(L-prolyl)pyrrolidine hydrochloride (1.18 g) as obtained by treating (2S)-1-[N-(t-butoxycarbonyl)-L-prolyl]-2-[1-hydroxy-2-(4-methoxyphenoxy) ethyl]pyrrolidine obtained in Example 25-A) with 4N hydrochloric acid-dioxane, there was obtained 661 mg of the title compound.

B) (2S)-1-[N-(4-Methoxybenzylaminocarbonyl)-L-prolyl]-2-(4-methoxyphenoxyacetyl)pyrrolidine

The title compound (387 mg) was obtained as colorless needles by oxidating (2S)-2-[1-hydroxy-2-(4-methoxyphenoxy)ethyl]-1-[N-(4-methoxybenzylaminocarbonyl)-L-prolyl]pyrrolidine (614 mg) using sulfur trioxide-pyridine complex (0.37 g) as in Example 25-C) (See Table 7).

The physiochemical properties of Compounds 1 to 28 are shown in Tables 1 to 7.

Table 1

| Comp. No. | W-(CH$_2$)n-A-CO-B- | X | R | Properties Melting point Optical rotation ($CH_3OH$) | EI-MS (m/z) | $^1$H NMR ($CDCl_3$, δ value) |
|---|---|---|---|---|---|---|
| 1 | 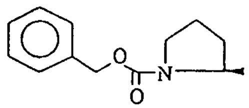 | S | 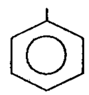 | colorless oily substance | 452(M+), 232,204,160, 91 | 1.7~2.2(8H,m),3.35~3.85(4H,m),3.81,3.86,3.93& 3.98(total 2H,d each,J=15.2Hz),4.40&4.52(total 1H,dd each,J=8.0,4.4Hz),4.54&4.82(total 1H,t each,J=6.3Hz),4.97,5.04,5.13&5.19(total 2H,d each,J=12.4Hz),7.2~7.4(10H,m) |
| 2 | 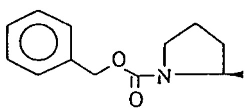 | SO | 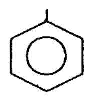 | colorless oily substance | 469(MH+), 451(MH+-$H_2O$), 232,204,160, 91 | 1.7~2.2(8H,m),3.35~3.85(4H,m),3.85,3.88,3.92, 4.00,4.01,4.07&4.11(total 2H,d each,J=14.9Hz), 4.3~4.75(total 2H,m),4.96,5.03,5.04,5.10,5.12, 5.17&5.18(total 2H,d each,J=12.4Hz),7.30(5H,m), 7.52(3H,m),7.66(2H,m) |
| 3 | 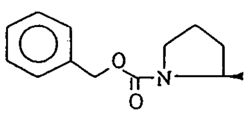 | $SO_2$ | 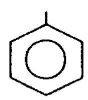 | colorless oily substance | 485(MH+), 232,204,160, 91 | 1.7~2.2(8H,m),3.35~3.90(4H,m),4.19,4.24,4.56& 4.62(total 2H,d each,J=13.8Hz),4.39,4.49&4.73 (total 2H,m),4.95,5.03,5.14&5.18(total 2H,d each, J=12.4Hz),7.35(5H,m),7.57(2H,m),7.67(1H,m), 7.89(2H,m) |
| 4 | 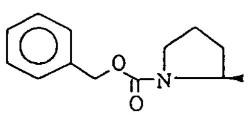 | O | 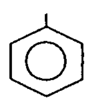 | colorless oily substance | 437(MH+), 302,232,204, 160,107,94,91 | 1.7~2.2(8H,m),3.4~3.9(4H,m),4.43&4.56(1H,dd each, J=8.1, 3.6Hz),4.66&4.93 (1H,dd each, J=8.7,5.7Hz),4.77,4.79&4.85(total 2H,s,d,d,J=16.9Hz),4.98,5.05,5.14&5.21(total 2H,d each, J=12.4Hz),6.98(3H,m),7.2~7.4(7H,m) |

Table 2

| Comp. No. | W-$(CH_2)$n-A-CO-B- | X | R | Properties Melting point Optical rotation($CH_3OH$) | EI-MS (m/z) | $^1$H NMR ($CDCl_3$, δ value) |
|---|---|---|---|---|---|---|
| 5 | 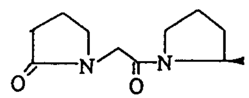 | SO | 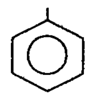 | colorless oily substance | 460(MH+), 444,292,223, 195,126,98, 70 | 1.8~2.2(10H,m),2.40(2H,m),3.3~4.7(12H,m), 7.52(3H,m),7.66(2H,m) |
| 6 | 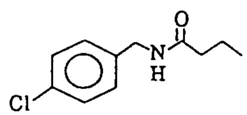 | SO | 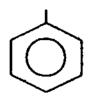 | colorless oily substance | 461(MH+), 445,224,218, 140,125,99, 78,70 | 1.8~2.1(4H,m),2.5~2.7(4H,m),3.58,(2H,m),3.83(1H,d, J=17.0Hz),3.99&4.04(total 1H,d each,J= 17.0Hz), 4.28 (1H,d,J=5.9Hz),4.37&4.38(total 1H,d each,J=5.9Hz), 4.50(1H,m),6.41(1H,m),7.1~7.3(4H,m),7.50(3H,m), 7.65(2H,m) |
| 7 | 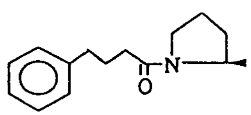 | SO | 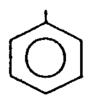 | colorless oily substance | 481(MH+), 463(MH+-$H_2O$), 376,355,313 244,216,147, 91 | 1.7~2.35(12H,m),2.65(2H,t,J=7.0Hz),3.40,3.56&3.88 (total 4H,m),3.85,4.01&4.07(total 2H,d,J= 14.8Hz,s, d,J=14.8Hz),4.55~4.70(2H,m),7.17(3H,m),7.26(2H,m), 7.51(3H,m),7.66(2H,m) |
| 8 | 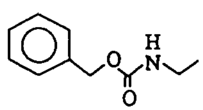 | SO | 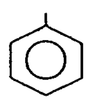 | colorless oily substance | 428(M+), 412,303,261, 195,160,153, 125,91 | 1.9~2.1(4H,m),3.45~3.60(2H,m),3.85~4.10(4H,m),4.55& 4.65(total 1H,dd each,J=7.0,5.0Hz),5.09&5.11(total 2H, s each),5.62(1H,br.s),7.34(5H, m),7.53(3H,m),7.68(2H,m) |

Table 3

| Comp. No. | W-$(CH_2)$n-A-CO-B- | X | R | Properties Melting point Optical rotation($CH_3OH$) | EI-MS (m/z) | $^1H$ NMR ($CDCl_3$, δ value) |
|---|---|---|---|---|---|---|
| 9 | 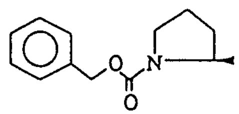 | SO | 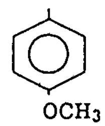 | colorless oily substance | 499(MH+), 483,344,302, 232,204,160, 155,139,91 | 1.8~2.2(8H,m),3.3~3.7(4H,m),3.83&3.85(3H,s each), 3.75~4.13(2H),4.35~4.74(2H,m),4.94~5.20(2H),7.02(2H, m),7.34(5H,m),7.60(2H,m) |
| 10 | 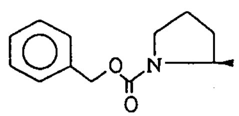 | SO | 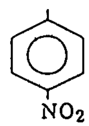 | colorless oily substance | 514(MH+), 497,379,344, 309,302,233, 204,171,160,91 | 1.8~2.2(8H,m),3.4~5.2(10H,m),7.35,(5H,m),7.88(2H,m), 8.39(2H,m) |
| 11 | 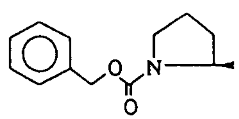 | SO | -$CH(CH_3)_2$ | colorless oily substance | 434(M+), 418,392,343,301, 232,204,160,91 | 1.31(6H,m),1.8~2.2(8H,m),2.96(1H,m),3.3~3.95(6H,m), 4.35~4.8(2H,m),4.95~5.2(2H),7.35(5H,m) |
| 12 | 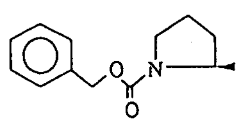 | 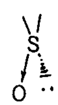 | 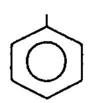 | colorless oily substance $[\alpha]_D$ − 177° | ——— | 1.7~2.2(8H,m),3.37,3.50,3.60&3.80(total 4H,m each), 3.85,3.89,4.06&4.10(total 2H,d each,J=14.9Hz), 4.33&4.60(total 1H,dd each,J=8.2,4.6Hz),4.41&4.52(total 1H,dd each,J=8.3,3.6Hz),7.30(5H,m),7.52(3H,m),7.70(2H,m) |

Table 4

| Comp. No. | W-$(CH_2)$n-A-CO-B- | X | R | Properties Melting point Optical rotation ($CH_3OH$) | EI-MS (m/z) | $^1$H NMR ($CDCl_3$, δ value) |
|---|---|---|---|---|---|---|
| 13 | 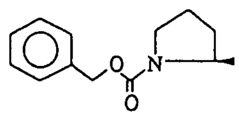 | 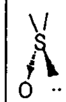 | 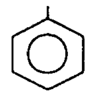 | colorless needles mp 120~121°C $[\alpha]_D$ − 23.4° | ———— | 1.7~2.2(8H,m),3.35~3.65&3.80(total 4H,m),3.94,4.01&4.05 (total 2H,d,d,s,J=13.4Hz),4.39&4.51(total 1H,dd each, J=8.1,3.3Hz),4.46&4.73(total 1H,dd each,J=7.5,5.7Hz), 4.97,5.03,5.12&5.17(total 2H,d each,J=12.4Hz), 7.34(5H,m),7.51(3H,m),7.69(2H,m) |
| 14 | 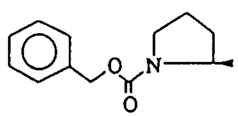 | 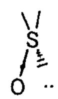 | 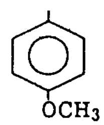 | colorless oily substance $[\alpha]_D$ − 183° | 498(M+), 482,391,232, 204,160,155, 139,91 | 1.7~2.2(8H,m),3.35~3.65&3.77(total 4H,m),3.80&3.84(total 1H,d each,J=14.7Hz),3.86(3H,s),4.07&4.11(total 1H,d each, J=14.7Hz),4.33&4.60(total 1H,dd each,J=8.0, 4.8Hz), 4.41&4.52(total 1H,dd each,J=8.3,3.8Hz),4.96,5.03, 5.10&5.17(total 2H, d each,J=12.4Hz),7.02(2H,m),7.23~7.35 (5H,m),7.62(2H,m) |
| 15 | 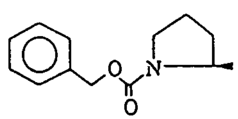 | 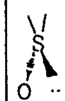 | 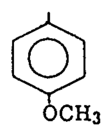 | colorless oily substance $[\alpha]_D$ − 61.2° | 498(M+), 482,391,343,301, 232,204,160,155, 139,91 | 1.7~2.2(8H,m),3.35~3.65&3.79(total 4H,m),3.85(3H,s), 3.95,4.00&4.04(total 2H,s,d,d,J=14.0 Hz),4.40&4.51(total 1H,dd each,J=8.2,4.0Hz),4.46&4.72(total 1H,dd each,J=7.6,5.5Hz),4.96,5.04,5.11&5.18(total 1H,d each, J=12.3Hz),7.02(2H,m),7.27~7.37(5H,m),7.61(2H,m) |
| 16 | 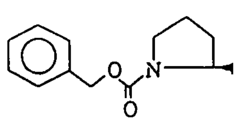 | 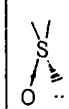 | 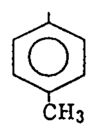 | colorless oily substance $[\alpha]_D$ − 216° | 482(M+), 466,343,301,232, 204,160,139,91 | 1.7~2.2(8H,m),2.42(3H,s),3.36,3.50,3.61&3.79(total 4H,m), 3.80&3.84(total 1H,d each,J=14.8Hz),4.05&4.09(total 1H, d each,J=14.8Hz),4.33&4.60(total 1H,dd each,J=7.9, 4.7Hz),4.41&4.52(total 1H,dd each,J=8.3,3.7Hz),4.96,5.03, 5.10&5.17(total 2H,d each,J=12.2Hz),7.28(2H,m), 7.33(5H,m),7.55(2H,m) |

Table 5

| Comp. No. | W-$(CH_2)$n-A-CO-B- | X | R | Properties Melting point Optical rotation ($CH_3OH$) | EI-MS (m/z) | $^1$H NMR ($CDCl_3$, δvalue) |
|---|---|---|---|---|---|---|
| 17 | 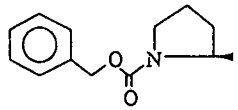 | 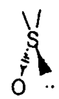 | 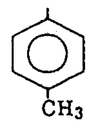 | colorless oily substance [α]$_D$ −30.0° | 482(M+), 466,343,301, 232,204,160, 139,91 | 1.7~2.2(8H,m),2.41(3H,s),3.35~3.65&3.81(total 4H,m), 3.90,3.97,3.98&4.04(total 2H,d each,J=14.0Hz), 4.39&4.51(total 1H,dd each,J=8.2,3.9Hz),4.46&4.73 (total 1H,dd each,J=7.8,5.7Hz),4.96,5.03,5.11&5.18 (total 2H,d each,J=12.4 Hz),7.27~7.35(7H,m), 7.54(2H,m) |
| 18 | 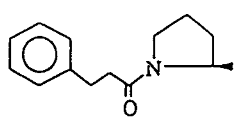 | 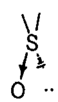 | 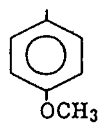 | colorless oily substance [α]$_D$ −192° | 496(M+), 480,341,299, 230,202,155 | 1.8~2.2(8H,m),2.61(2H,m),2.94(2H,t,J=8.2Hz), 3.40,3.60&3.88(total 4H,m),3.82(1H,d,J=14.9Hz), 3.85(3H,s),4.10(1H,d,J=14.9Hz),4.59(1H,dd,J=8.2,5.0Hz), 4.64(1H,dd,J=7.7,3.7Hz),7.01(2H,m),7.19(2H,m),7.28(3H,m), 7.62(2H,m), |
| 19 | 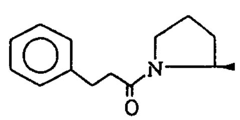 | 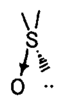 | 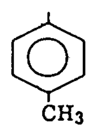 | colorless needles mp 127~129°C [α]$_D$ −235° | 480(M+), 464,341,299,230, 202,139 | 1.8~2.2(8H,m),2.42(3H,s),2.58(2H,m),2.94(2H,t,J=8.2Hz), 3.41,3.62&3.90(total 4H,m),3.83(1H,d,J=14.9Hz), 4.08(1H,d,J=14.9Hz),4.59(1H,dd,J=8.1,4.9Hz),4.64(1H,dd, J=7.7,3.6Hz),7.18~7.34(7H,m),7.55(2H,m) |
| 20 | 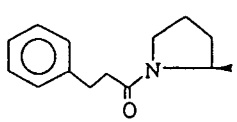 | 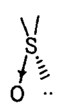 | 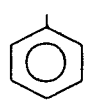 | colorless needles mp 163~166°C [α]$_D$ −231° | 466(M+), 450,341,299,230, 202,125 | 1.8~2.2(8H,m),2.60(2H,m),2.94(2H,t,J=8.1Hz), 3.42,3.61&3.85(total 4H,m),3.86(1H,d,J=15.0Hz), 4.09(1H,d,J=15.0Hz),4.59(1H,dd,J=8.0,4.9Hz),4.64(1H,dd, J=7.7,3.8Hz),7.16~7.34(5H,m),7.53(3H,m),7.66(2H,m) |

Table 6

| Comp. No. | W-(CH2)n-A-CO-B- | X | R | Properties Melting point Optical rotation($CH_3OH$) | EI-MS (m/z) | $^1H$ NMR ($CDCl_3$, δ value) |
|---|---|---|---|---|---|---|
| 21 | 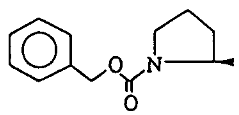 | O | 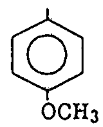 | colorless oily substance [α]D − 41.9° | 466(M+), 343,301,232, 204,160,91 | 1.8~2.2(8H,m),3.4~3.9(4H,m),3.76&3.77(total 3H,s each), 4.45,4.55,4.67&4.91(total 2H,m),4.72,4.74&4.79(total 2H,s,d,d,J=16.9Hz),4.97,5.04,5.13&5.19(total 2H,d each, J=12.4Hz),6.83(4H,m),7.27~7.37(5H,m) |
| 22 | 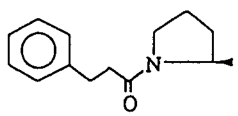 | O | 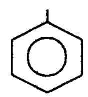 | colorless needles mp 122.8~124°C [α]D − 116° | 434(M+), 341,299,230, 202 | 1.8~2.2(8H,m),2.61(2H,m),2.95(2H,m),3.35~3.70&4.06(total 4H,m),4.62(1H,dd,J=7.6,5.3Hz),4.68(1H,dd,J=8.1,4.2Hz), 4.84(1H,d,J=18.0Hz),4.98(1H,d,J=18.0Hz),6.90~7.32(10H, m) |
| 23 | 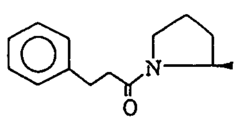 | O | 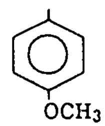 | colorless needles | 464(M+), 341,299,230, 202 | 1.85~2.2(8H,m),2.61(2H,m),2.94(2H,m),3.35~3.70&4.05 (total 4H,m),3.66(3H,s),4.61(1H,dd,J=7.7,5.3Hz),4.68(1H, dd,J=7.0,5.3Hz),4.80(1H,d,J=17.9Hz),4.92(1H,d, J=17.9Hz),6.78~7.29(9H,m) |
| 24 | 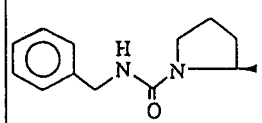 | O | 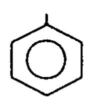 | colorless particles mp 138.1~140.4°C | 435(M+), 300,231,203, 91 | 1.8~2.3(8H,m),3.32(1H,m),3.51(1H,m),3.62(1H,m),3.90(1H, m),4.32(1H,dd,J=14.5,4.9Hz),4.52(1H,dd,J=14.5,6.0Hz), 4.63(1H,t,J=5.4Hz),4.70(1H,dd,J=7.8,3.2Hz),4.78(1H,d,J= 17.0Hz),4.85(1H,d,J=17.0Hz),4.90(1H,dd,J=7.9,5.4Hz), 6.9~7.0(3H,m),7.25~7.35(7H,m) |

Table 7

| Comp. No. | W-(CH2)n-A-CO-B- | X | R | Properties Melting point Optical rotation($CH_3OH$) | EI-MS (m/z) | ¹H NMR (CDCl3, δvalue) |
|---|---|---|---|---|---|---|
| 25 | 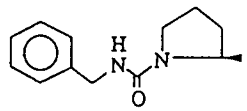 | O | 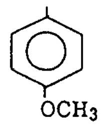 | colorless needles mp 127.7~129.1°C [α]D − 48.9° | CI-MS; 466(MH+), 315,231,125, 91 | 1.8~2.3(8H,m),3.32(1H,m),3.51(1H,m),3.62(1H,m),3.76(3H,s),3.90(1H,m),4.31(1H,dd,J=14.6,5.0Hz),4.53(1H,dd,J=14.6,6.2Hz),4.68(2H,m),4.72(1H,d,J=16.8Hz),4.79(1H,d,J=16.8Hz),4.86(1H,dd,J=8.3,5.3Hz),6.8~6.9(4H,m),7.2~7.4(5H,m) |
| 26 | 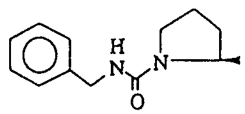 | 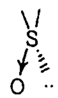 | 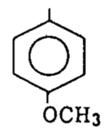 | colorless needles mp 140~142°C [α]D − 213° | 497(M+), 342,231,203, 155,91 | 1.9~2.3(8H,m),3.31(1H,m),3.47(1H,m),3.61(1H,m),3.83(1H,d,J=14.8Hz),3.86(3H,s),3.89(1H,m),4.10(1H,d,J=14.8Hz),4.31(1H,dd,J=14.6,5.0Hz),4.50(1H,dd,J=14.6,6.0Hz),4.55~4.7(3H,m),7.03(2H,d,J=8.9Hz),7.25~7.35(5H,m),7.61(2H,d,J=8.9Hz) |
| 27 | 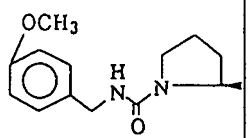 | 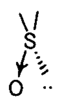 | 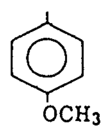 | colorless needles mp 165.5~167.5°C [α]D − 192° | 527(M+), 261,233,155, 121 | 1.9~2.2(8H,m),3.29(1H,m),3.46(1H,m),3.61(1H,m),3.78(3H,s),3.83(1H,d,J=14.8Hz),3.86(3H,s),3.89(1H,m),4.10(1H,d,J=14.8Hz),4.24(1H,br d,J=14.2Hz),4.42(1H,br d,J=14.2Hz),4.53(1H,br s),4.59(1H,dd,J=8.0,5.0Hz),4.67(1H,dd,J=7.7,3.2Hz),6.84(2H,d,J=8.6Hz),7.02(2H,d,J=8.8Hz),7.22(2H,d,J=8.6Hz),7.61(2H,d,J=8.8Hz) |
| 28 | 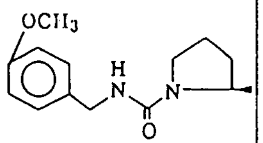 | O | 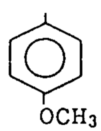 | colorless needles mp 130.2~132.5°C [α]D − 43.9° | FAB-MS; 496(MH+), 372,359 | 1.8~2.3(8H,m),3.30(1H,m),3.48(1H,m),3.62(1H,m),3.76(3H,s),3.79(3H,s),3.91(1H,m),4.25(1H,dd,J=14.2,4.8Hz),4.44(1H,dd,J=14.2,5.9Hz),4.54(1H,br t,J=5.4Hz),4.69(1H,dd,J=7.8,3.2Hz),4.73(1H,d,J=16.8Hz),4.80(1H,d,J=16.8Hz),4.89(1H,dd,J=8.4,5.7Hz),6.8~6.9(6H,m),7.25(2H,d,J=8.6Hz) |

Note that the present invention is not limited to these examples, and Compounds 29-44 shown in Table 8, for example, are also encompassed in the present invention.

Table 8

| Comp. No. | Formula | Comp. No. | Formula |
|---|---|---|---|
| 29 | 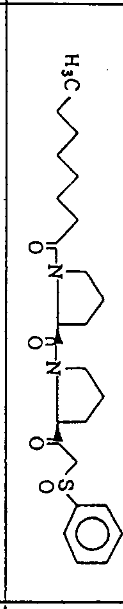 | 37 | 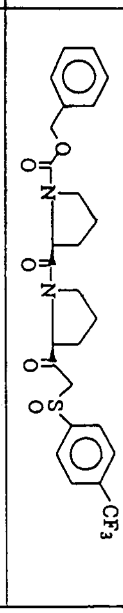 |
| 30 | 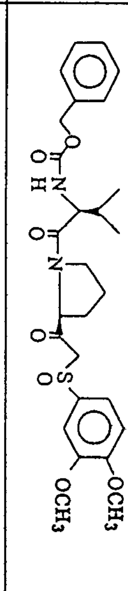 | 38 | 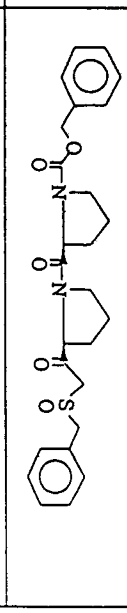 |
| 31 | 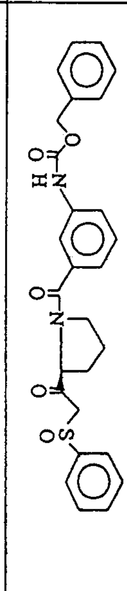 | 39 | 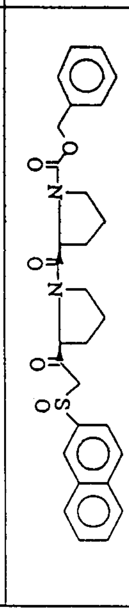 |
| 32 | 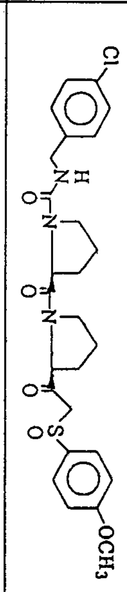 | 40 | 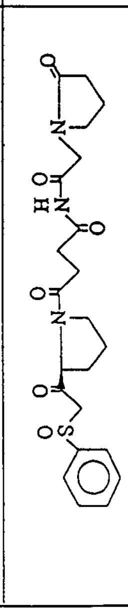 |
| 33 | 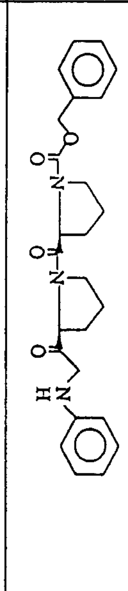 | 41 | 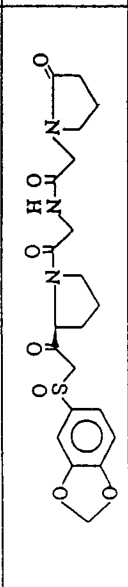 |
| 34 | 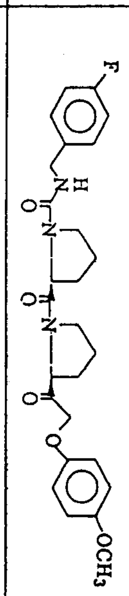 | 42 | 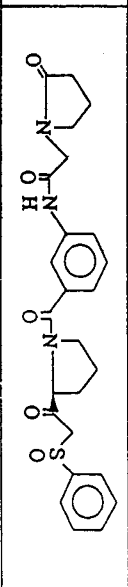 |
| 35 | 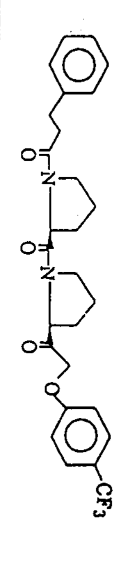 | 43 | 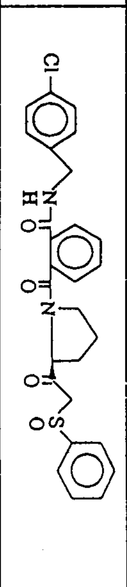 |
| 36 | 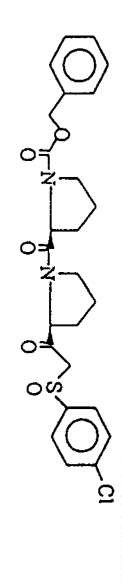 | 44 | 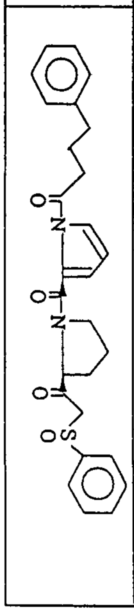 |

The proline derivatives of the present invention represented by the formula (I) mentioned above were examined for the *in vitro* prolyl endopeptidase-inhibitory activity and specificity in inhibitory activities against various endopeptidases.

Experiment Example 1

Prolyl endopeptidase-inhibitory activity

A mixture of a 0.1 M potassium sodium phosphate buffer (pH 7.0) (2675 μl), a solution of the compounds of the present invention in a 0.1 M potassium sodium phosphate buffer (pH 7.0) (100 μl) and a solution of prolyl endopeptidase extracted from rat brain in a 25 mM sodium phosphate buffer (100 μl) [123 unit/l, pH 6.8, containing 1 mM dithiothreitol and 0.5 mM EDTA, prepared by the method described in J.

Neurochem., *35*, 527 (1980)] was preincubated at 30°C for 30 minutes. Thereto was added a 0.2 mM solution of 7-(N-succinyl-glycyl-prolyl)-4-methylcoumarinamide (Peptide Institute, INC.) in a 0.1 M potassium sodium phosphate buffer (pH 7.0) (125 µl), and the mixture was incubated at 30°C for 1 hour. The reaction mixture was immersed in ice (0°C) to terminate the reaction, and the fluorescence ($a_1$) was determined ten minutes later (excitation at 370 nm and emission at 440 nm). Concurrently, there were conducted the experiment wherein, in the above system, the prolyl endopeptidase solution was substituted for a 25 mM sodium phosphate buffer (pH 6.8, containing 1 mM dithiothreitol and 0.5 mM EDTA) and the experiment wherein the solution of the compounds of the present invention was also substituted for a 0.1 M potassium sodium phosphate buffer (pH 7.0). Each fluorescence ($a_2$ and $a_3$) was also determined respectively [See *Tanpakushitsu Kakusan Koso*, *29*, 127 (1984)]. The prolyl endopeptidase-inhibition rate was calculated by the following formula, and $IC_{50}$, a concentration of a compound producing 50% inhibition, was estimated by semilogarithmic graph paper. The results are summarized in Table 9.

$$\text{Percent inhibition (\%)} = \left(1 - \frac{a_1 - a_2}{a_3 - a_2}\right) \times 100$$

Table 9

| Compound No. | $IC_{50}$ (nM) | Compound No. | $IC_{50}$ (nM) |
|---|---|---|---|
| 1 | 0.3 | 15 | 20 |
| 2 | 1.2 | 16 | 0.3 |
| 3 | 12 | 17 | 1.5 |
| 4 | 0.3 | 18 | 2.5 |
| 5 | 55 | 19 | 1.5 |
| 6 | 100 | 20 | 0.5 |
| 7 | 0.3 | 21 | 1.5 |
| 8 | 40 | 22 | 15 |
| 9 | 0.65 | 23 | 40 |
| 10 | 1.0 | 24 | 15 |
| 11 | 3.0 | 25 | 25 |
| 12 | 0.2 | 26 | 15 |
| 13 | 80 | 27 | 0.5 |
| 14 | 1.5 | 28 | 2.5 |

Experiment Example 2

Inhibitory activity against various proteases

The compounds of the present invention were examined for specificity in inhibitory activities against

various proteases. As a result, it was found that the compounds of the present invention specifically inhibited only prolyl endoprotease, as shown in the following Table 10.

Table 10-a

| Compound No. | Concentration (µM) | prolyl endo-peptidase | trypsin | chymotrypsin | leucine amino-peptidase | elastase | cathepsin B |
|---|---|---|---|---|---|---|---|
| 1 | 0.1 | 90 | 0 | 20 | 0 | 0 | 0 |
| 2 | 0.1 | 100 | 0 | 0 | 0 | 0 | 0 |
| 3 | 0.1 | 91 | 0 | 0 | 0 | 0 | 0 |
| 4 | 0.1 | 98 | 0 | 0 | 0 | 0 | 0 |
| 5 | 10 | 92 | 0 | 0 | 0 | 0 | 90 |
| 6 | 10 | 85 | 0 | 0 | 0 | 0 | 60 |
| 7 | 0.1 | 98 | 0 | 0 | 0 | 0 | 0 |
| 8 | 1 | 98 | 0 | 0 | 0 | 0 | 20 |
| 9 | 0.1 | 98 | 0 | 0 | 0 | 0 | 20 |
| 10 | 0.1 | 97 | 0 | 0 | 0 | 0 | 20 |
| 11 | 0.1 | 92 | 0 | 0 | 0 | 0 | 0 |
| 12 | 0.1 | 98 | 0 | 0 | 0 | 0 | 0 |
| 13 | 0.1 | 97 | 0 | 0 | 0 | 0 | 0 |
| 14 | 0.1 | 90 | 0 | 0 | 0 | 0 | 0 |

Table 10-b

| Compound No. | Concentration (μM) | prolyl endopeptidase | trypsin | chymotrypsin | leucine aminopeptidase | elastase | cathepsin B |
|---|---|---|---|---|---|---|---|
| 15 | 1 | 92 | 0 | 0 | 0 | 0 | 0 |
| 16 | 0.1 | 98 | 0 | 0 | 0 | 0 | 0 |
| 17 | 0.1 | 97 | 0 | 0 | 0 | 0 | 0 |
| 18 | 0.1 | 98 | 0 | 0 | 0 | 0 | 0 |
| 19 | 0.1 | 99 | 0 | 0 | 0 | 0 | 0 |
| 20 | 0.1 | 99 | 0 | 0 | 0 | 0 | 0 |
| 21 | 0.1 | 93 | 0 | 0 | 0 | 0 | 0 |
| 22 | 1 | 98 | 0 | 0 | 0 | 0 | 0 |
| 23 | 1 | 96 | 0 | 0 | 0 | 0 | 0 |
| 24 | 1 | 99 | 0 | 0 | 0 | 0 | 0 |
| 25 | 1 | 97 | 0 | 0 | 0 | 0 | 0 |
| 26 | 1 | 99 | 0 | 0 | 0 | 0 | 0 |
| 27 | 0.1 | 99 | 0 | 0 | 0 | 0 | 0 |
| 28 | 0.1 | 97 | 0 | 0 | 0 | 0 | 0 |

The methods for the measurement of inhibitory activities against various proteases and the method for calculating the inhibition rate are as follows.

Determination of trypsin-inhibitory activity

In the present invention, a 50 mM Tris-HCl buffer (pH 8.0) was employed as a buffer for measurement.

To a mixture of the above-mentioned buffer (850 μl), a solution of the compounds of the present invention in the same buffer (50 μl) and a 0.02 μM solution of trypsin (derived from bovine pancreas, Sigma) in the same buffer (50 μl) was added a 200 μM solution of 7-(prolyl-phenylaranyl-arginyl)-4-methylcoumarinamide (Peptide Institute, Inc.) in the same buffer (50 μl),and the mixture was incubated at 30 °C for 1 hour. The reaction mixture was immersed in ice (0 °C) to terminate the reaction, and the

fluorescence ($b_1$) was determined 1 hour later (excitation at 370 nm and emission at 440 nm). Concurrently, there were conducted the experiment wherein, in the system above, the trypsin solution was substituted for a buffer alone, and the experiment wherein the solution of the compounds of the present invention was also substituted for a buffer above. Each fluorescence ($b_2$ and $b_3$) was also determined respectively.

Determination of chymotrypsin-inhibitory activity

Similarly, by following the procedure mentioned above, but employing a 50 mM Tris-HCl buffer (pH 8.0) as a buffer for measurement, a 0.2 $\mu$M solution of chymotrypsin (derived from bovine pancreas, Sigma) in the same buffer as an enzyme solution and a 200 $\mu$M solution of 7-(N-succinyl-leucyl-leucyl-valyl-tyrosyl)-4-methylcoumarinamide (Peptide Institute, Inc.) in the same buffer as a substrate solution. Each fluorescence ($c_1$, $c_2$ and $c_3$) was also determined, respectively.

Determination of leucine aminopeptidase-inhibitory activity

Similarly, by following the procedure mentioned above, but employing a 50 mM Tris-HCl buffer (pH 8.0) as a buffer for measurement, a 0.2 $\mu$M solution of leucine aminopeptidase (derived from swine kidney, Sigma) in the same buffer as an enzyme solution and a 200 $\mu$M solution of 7-leucyl-4-methylcoumarinamide (Peptide Institute, Inc.) in the same buffer as a substrate solution. Each fluorescence ($d_1$, $d_2$ and $d_3$) was also determined, respectively.

Determination of elastase-inhibitory activity

Similarly, by following the procedure mentioned above, but employing a 1 mM Tris-HCl buffer (pH 8.5) as a buffer for measurement, a 0.2 $\mu$M solution of elastase (derived from swine pancreas, Sigma) in the same buffer as an enzyme solution and a 200 $\mu$M solution of 7-(N-succinyl-aranyl-prolyl-aranyl)-4-methylcoumarinamide (Peptide Institute, Inc.) in the same buffer as a substrate solution. Each fluorescence ($e_1$, $e_2$ and $e_3$) was also determined, respectively.

Determination of cathepsin B-inhibitory activity

Similarly, by following the procedure mentioned above, but employing a 100 mM sodium phosphate buffer (pH 6.0; containing 1.33 mM EDTA $Na_2$) as a buffer for measurement, a 0.02 $\mu$M solution of cathepsin B (derived from bovine spleen, Sigma) in the same buffer as an enzyme solution and a 200 $\mu$M solution of 7-(N-benzyloxycarbonyl-phenylaranyl-arginyl)-4-methylcoumarinamide (Peptide Institute, Inc.) in the same buffer as a substrate solution. Each fluorescence ($f_1$, $f_2$ and $f_3$) was also determined, respectively.

Using the fluorescence $x_1$, $x_2$ and $x_3$ measured in the above-mentioned manner, the inhibitory rate against various proteases was calculated by the following formula where x stands for b, c, d, e or f:

$$\text{Percent inhibition (\%)} = \left(1 - \frac{x_1 - x_2}{x_3 - x_2}\right) \times 100$$

The novel proline derivatives of the formula (I) of the present invention exhibit very strong prolyl endopeptidase-inhibitory activities, while they exhibit no or very weak inhibitory activity against proteases such as trypsin, chymotrypsin, leucine aminopeptidase, elastase and cathepsin B, which fact shows that the compounds of the present invention specifically inhibit decomposition and inactivation of endogeneous peptides containing proline residues, which are intracerebral hormones, neurotransmitters and other peptides which are considered to be concerned with learning and memory process, such as TRH, substance P, neurotensin, vasopressin, or the like.

Based on such characteristic properties, the compounds of the present invention are expected to contribute to the improvement of the symptoms of various diseases concerned with hormones and neurotransmitters, and in addition, can be used for the prevention and/or treatment of dementia and amnesia as agents which act directly on the central symptoms of dementia.

**Claims**

**1.** A proline derivative of the following formula

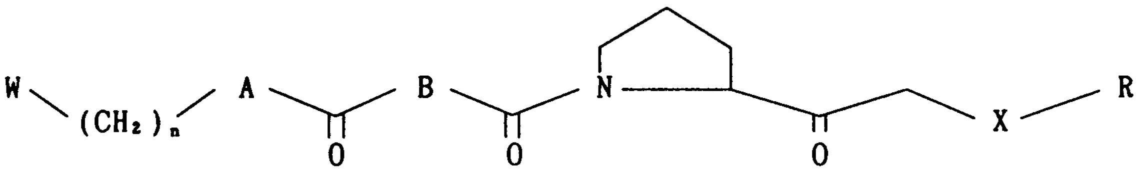

wherein:

A is -O-, -NH-,

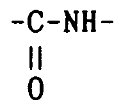

or absent;

B is

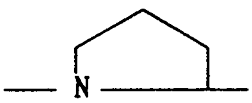, 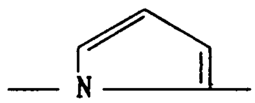, 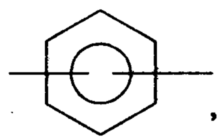, 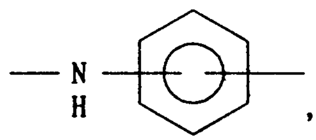,

$-(CH_2)_k-$ or

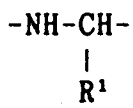

where k is an integer of 1 to 3, $R^1$ is hydrogen atom or a lower alkyl having 1 to 5 carbon atoms;

W is

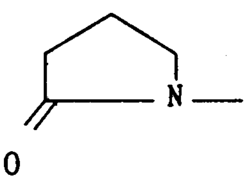, 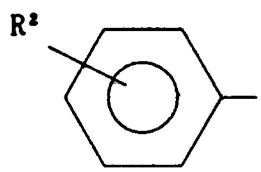

or $CH_3$- where $R^2$ is hydrogen atom, halogen atom or a lower alkoxy;

X is -S-, -SO-, $-SO_2-$, -O- or -NH-;

R is

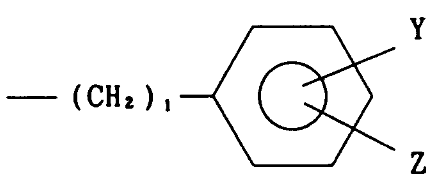

or a lower alkyl having 1 to 5 carbon atoms where 1 is an integer of 0 to 3, Y and Z are the same or

different and each is hydrogen atom, halogen atom, a lower alkyl having 1 to 5 carbon atoms which may be substituted by fluorine atom, amino, nitro, hydroxyl or a lower alkoxy, and Y and Z may combinedly form a saturated or unsaturated 5- or 6-membered ring; and
n is an integer of 1 to 6.

**2.** A proline derivative according to Claim 1, which is selected from the group consisting of (2S)-1-(N-benzyloxycarbonyl-L-prolyl)-2-[(phenylthio)acetyl]pyrrolidine, (2S)-1-(N-benzyloxycarbonyl-L-prolyl)-2-[-(phenylsulfinyl)acetyl]pyrrolidine, (2S)-1-(N-benzyloxycarbonyl-L-prolyl)-2-[(phenylsulfonyl)acetyl]-pyrrolidine, (2S)-1-(N-benzyloxycarbonyl-L-prolyl)-2-(phenoxyacetyl)pyrrolidine, (2S)-1-{N-[(2-oxo-1-pyrrolidinyl)acetyl]-L-prolyl}-2-[(phenylsulfinyl)acetyl]pyrrolidine, (2S)-1-[4-(4-chlorobenzylamino)-4-oxobutyryl]-2-[(phenylsulfinyl)acetyl]pyrrolidine, (2S)-1-[N-(4-phenylbutyryl)-L-prolyl]-2-[(phenylsulfinyl)-acetyl]pyrrolidine, (2S)-1-[N-(benzyloxycarbonyl)glycyl]-2-[(phenylsulfinyl)acetyl]pyrrolidine, (2S)-1-(N-benzyloxycarbonyl-L-prolyl)-2-[(4-methoxyphenylsulfinyl)acetyl]pyrrolidine, (2S)-1-(N-benzyloxycarbonyl-L-prolyl)-2-[(4-nitrophenylsulfinyl)acetyl]pyrrolidine, (2S)-1-(N-benzyloxycarbonyl-L-prolyl)-2-[(isopropylsulfinyl)acetyl]pyrrolidine, (2S)-1-(N-benzyloxycarbonyl-L-prolyl)-2-{[(S)-phenylsulfinyl]acetyl}pyrrolidine, (2S)-1-(N-benzyloxycarbonyl-L-prolyl)-2-{[(R)-phenylsulfinyl]-acetyl}pyrrolidine, (2S)-1-(N-benzyloxycarbonyl-L-prolyl)-2-{[(S)-4-methoxyphenylsulfinyl]-acetyl}pyrrolidine, (2S)-1-(N-benzyloxycarbonyl-L-prolyl)-2-{[(R)-4-methoxyphenylsulfinyl]-acetyl}pyrrolidine, (2S)-1-(N-benzyloxycarbonyl-L-prolyl)-2-{[(S)-p-tolylsulfinyl]acetyl}pyrrolidine, (2S)-1-(N-benzyloxycarbonyl-L-prolyl)-2-{[(R)-p-tolylsulfinyl]acetyl}pyrrolidine, (2S)-2-{[(S)-4-methoxyphenylsulfinyl]acetyl}-1-[N-(3-phenylpropionyl)-L-prolyl]pyrrolidine, (2S)-1-[N-(3-phenylpropionyl)-L-prolyl]-2-{[(S)-p-tolylsulfinyl]acetyl}pyrrolidine, (2S)-1-[N-(3-phenylpropionyl)-L-prolyl]-2-{[-(S)-phenylsulfinyl]acetyl}pyrrolidine, (2S)-1-(N-benzyloxycarbonyl-L-prolyl)-2-[(4-methoxyphenoxy)-acetyl]pyrrolidine, (2S)-2-(phenoxyacetyl)-1-[N-(3-phenylpropionyl)-L-prolyl]pyrrolidine, (2S)-2-[(4-methoxyphenoxy)acetyl]-1-[N-(3-phenylpropionyl)-L-prolyl]pyrrolidine, (2S)-1-(N-benzylaminocarbonyl-L-prolyl)-2-(phenoxyacetyl)pyrrolidine,(2S)-1-(N-benzylaminocarbonyl-L-prolyl)-2-(4-methoxyphenoxyacetyl)pyrrolidine, (2S)-1-(N-benzylaminocarbonyl-L-prolyl)-2-{[(S)-4-methoxyphenylsulfinyl]acetyl}pyrrolidine, (2S)-1-[N-(4-methoxybenzylaminocarbonyl)-L-prolyl]-2-{[(S)-4-methoxyphenylsulfinyl]acetyl}pyrrolidine and (2S)-1-[N-(4-methoxybenzylaminocarbonyl)-L-prolyl]-2-(4-methoxyphenoxyacetyl)pyrrolidine.

**3.** A method for production of a proline derivative of the formula

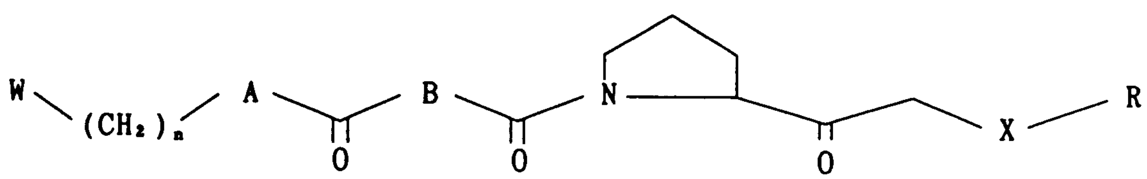

wherein W, A, B, X, R and n are as defined above, comprising subjecting a compound of the formula

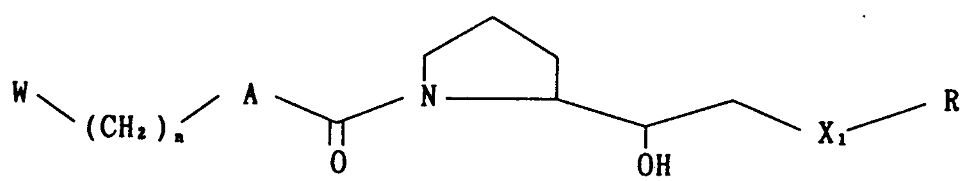

wherein $X_1$ is S, SO or $SO_2$, and W, A, R are as defined above, or a compound of the formula

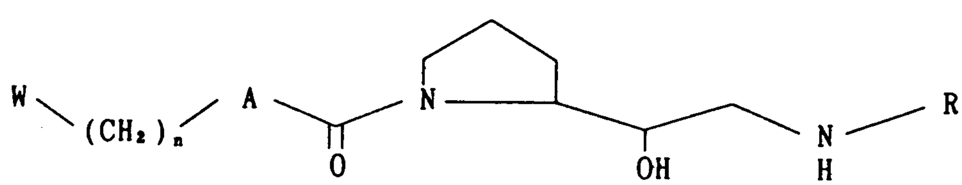

wherein W, A and R are as defined above, or an optically active compound of the formula

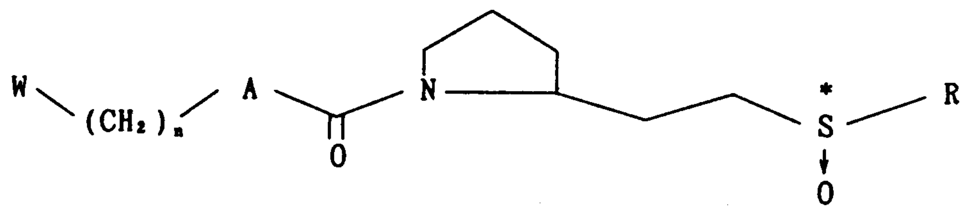

wherein W, A and R are as defined above, to oxidation; or reacting an optically active compound of the formula

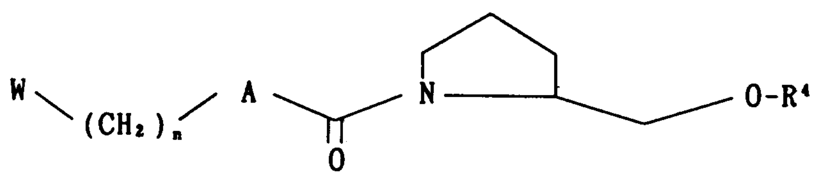

wherein $R^4$ is a carbonyl protecting group and W, A and R are as defined above, with a compound of the formula

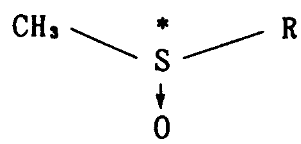

wherein R is as defined above.

**4.** A pharmaceutical composition containing an effective amount of a proline-derivative of the following formula

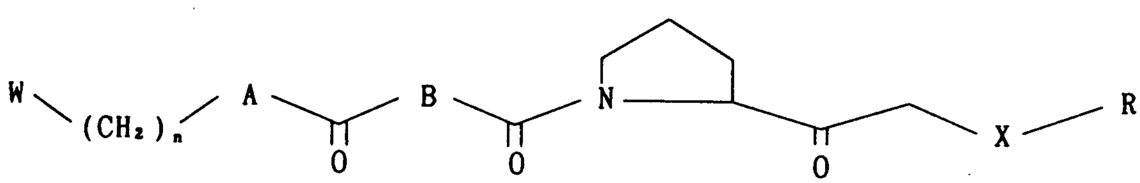

wherein:
A is -O-, -NH-,

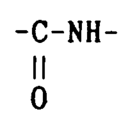

or absent;
B is

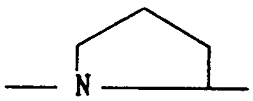, 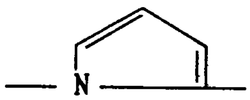, 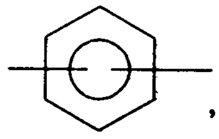, 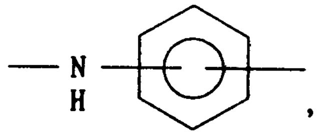,

$-(CH_2)_k-$ or

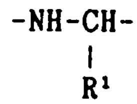

where k is an integer of 1 to 3, $R^1$ is hydrogen atom or a lower alkyl having 1 to 5 carbon atoms;
W is

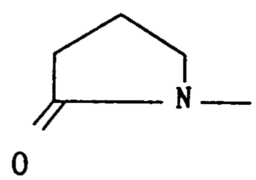, 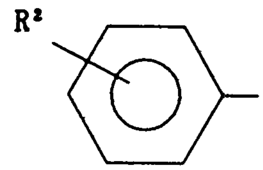

or $CH_3$- where $R^2$ is hydrogen atom, halogen atom or a lower alkoxy;
X is -S-, -SO-, $-SO_2-$, -O- or -NH-;
R is

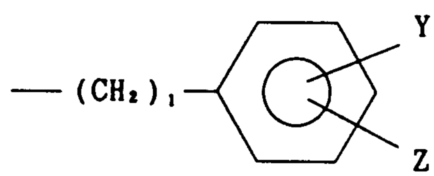

or a lower alkyl having 1 to 5 carbon atoms where 1 is an integer of 0 to 3, Y and Z are the same or different and each is hydrogen atom, halogen atom, a lower alkyl having 1 to 5 carbon atoms which may be substituted by fluorine atom, amino, nitro, hydroxyl or a lower alkoxy, and Y and Z may combinedly form a saturated or unsaturated 5- or 6-membered ring; and
n is an integer of 1 to 6, and a pharmaceutically acceptable diluent.

**5.** A pharmaceutical composition according to Claim 4, wherein the proline derivative is selected from the group consisting of (2S)-1-(N-benzyloxycarbonyl-L-prolyl)-2-[(phenylthio)acetyl]pyrrolidine, (2S)-1-(N-benzyloxycarbonyl-L-prolyl)-2-[(phenylsulfinyl)acetyl]pyrrolidine, (2S)-1-(N-benzyloxycarbonyl-L-prolyl)-2-[(phenylsulfonyl)acetyl]pyrrolidine, (2S)-1-(N-benzyloxycarbonyl-L-prolyl)-2-(phenoxyacetyl)pyrrolidine, (2S)-1-{N-[(2-oxo-1-pyrrolidinyl)acetyl]-L-prolyl}-2-[(phenylsulfinyl)acetyl]pyrrolidine, (2S)-1-[4-(4-chlorobenzylamino)-4-oxobutyryl]-2-[(phenylsulfinyl)acetyl]pyrrolidine, (2S)-1-[N-(4-phenylbutyryl)-L-prolyl]-2-[(phenylsulfinyl)acetyl]pyrrolidine, (2S)-1-[N-(benzyloxycarbonyl)glycyl]-2-[(phenylsulfinyl)-acetyl]pyrrolidine, (2S)-1-(N-benzyloxycarbonyl-L-prolyl)-2-[(4-methoxyphenylsulfinyl)acetyl]pyrrolidine, (2S)-1-(N-benzyloxycarbonyl-L-prolyl)-2-[(4-nitrophenylsulfinyl)acetyl]pyrrolidine, (2S)-1-(N-benzyloxycarbonyl-L-prolyl)-2-[(isopropylsulfinyl)acetyl]pyrrolidine, (2S)-1-(N-benzyloxycarbonyl-L-prolyl)-2-{[(S)-phenylsulfinyl]acetyl}pyrrolidine, (2S)-1-(N-benzyloxycarbonyl-L-prolyl)-2-{[(R)-phenylsulfinyl]acetyl} pyrrolidine, (2S)-1-(N-benzyloxycarbonyl-L-prolyl)-2-{[(S)-4-methoxyphenylsulfinyl]acetyl}pyrrolidine, (2S)-1-(N-benzyloxycarbonyl-L-prolyl)-2-{[(R)-4-methoxyphenylsulfinyl]acetyl}pyrrolidine, (2S)-1-(N-benzyloxycarbonyl-L-prolyl)-2-{[(S)-p-tolylsulfinyl]-acetyl}pyrrolidine, (2S)-1-(N-benzyloxycarbonyl-L-prolyl)-2-{[(R)-p-tolylsulfinyl]acetyl}pyrrolidine, (2S)-2-{[(S)-4-methoxyphenylsulfinyl]acetyl}-1-[N-(3-phenylpropionyl)-L-prolyl]pyrrolidine, (2S)-1-[N-(3-phenylpropionyl)-L-prolyl]-2-{[(S)-p-tolylsulfinyl]acetyl}pyrrolidine, (2S)-1-[N-(3-phenylpropionyl)-L-prolyl]-2-{[-(S)-phenylsulfinyl]acetyl}pyrrolidine, (2S)-1-(N-benzyloxycarbonyl-L-prolyl)-2-[(4-methoxyphenoxy)-acetyl]pyrrolidine, (2S)-2-(phenoxyacetyl)-1-[N-(3-phenylpropionyl)-L-prolyl]pyrrolidine, (2S)-2-[(4-methoxyphenoxy)acetyl]-1-[N-(3-phenylpropionyl)-L-prolyl]pyrrolidine, (2S)-1-(N-benzylaminocarbonyl-L-prolyl)-2-(phenoxyacetyl)pyrrolidine,(2S)-1-(N-benzylaminocarbonyl-L-prolyl)-2-(4-methoxyphenoxyacetyl)pyrrolidine, (2S)-1-(N-benzylaminocarbonyl-L-prolyl)-2-{[(S)-4-methoxyphenylsulfinyl]acetyl}pyrrolidine, (2S)-1-[N-(4-methoxybenzylaminocarbonyl)-L-prolyl]-2-{[(S)-4-methoxyphenylsulfinyl]acetyl}pyrrolidine and (2S)-1-[N-(4-methoxybenzylaminocarbonyl)-L-prolyl]-2-(4-methoxyphenoxyacetyl)pyrrolidine.